# EUROPEAN PATENT SPECIFICATION

(11) **EP 1 159 261 B1**
(45) Date of publication and mention of the grant of the patent: **02.11.2005**
(21) Application number: 00913365.3
(22) Date of filing: 04.02.2000
(51) Int. Cl.: C07C 279/00

(54) **ANTIZYME MODULATORS AND THEIR USE**
ANTIZYMMODULATOREN SOWIE DEREN VERWENDUNG
MODULATEURS D'ANTIZYME ET LEUR UTILISATION

(30) Priority: 05.02.1999 US 118892 P
(43) Date of publication of application: 05.12.2001
(73) Proprietor: ORIDIGM CORPORATION, Seattle, WA 98103 (US)
(72) Inventor: VERMEULIN, Nicolaas, M., J., Woodinville, WA 98072 (US); BURNS, Mark, R., Shoreline, WA 98177 (US); WEBB, Heather, K., Seattle, WA 98107 (US)
(74) Representative: W.P. Thompson & Co.
(86) International application number: PCT/US2000/002972
(87) International publication number: WO 2000/046187

(56) References cited:
- WO-A-99/03823
- US-A- 5 498 522
- US-A- 5 677 350
- CHEMICAL ABSTRACTS, vol. 129, no. 12, 21 September 1998 (1998-09-21) Columbus, Ohio, US; abstract no. 147272, SATRIANO, JOSEPH ET AL: "Agmatine suppresses proliferation by frameshift induction of antizyme and attenuation of cellular polyamine levels" XP002142375 & J. BIOL. CHEM. (1998), 273(25), 15313-15316 ,
- BEILSTEIN INFORMATION SERVICE FILE: XFIRE, XP002142374

## Description

### TECHNICAL FIELD

This invention in the fields of chemistry and biochemistry relates to the synthesis and use of novel agmatine and polyamine analogs with pharmacological or agricultural applications. Agmatine has been shown to be involved in the regulation of intracellular polyamine levels by inducing antizyme, which regulates both cellular polyamine biosynthesis and uptake/import. Previous pharmacological focus has been on the alternative of either inhibiting polyamine biosynthesis or inhibiting polyamine uptake/import to decrease intracellular polyamine levels. The instant invention provides agmatine and polyamine analogs that decrease cellular polyamine levels by both means but are not necessarily metabolized like agmatine and polyamines. As drugs, these analogs are used to treat disorders of undesired cell proliferation, primarily cancer, alone or combined with other agents, particularly other inhibitors of polyamine synthesis or transport. The invention also identifies key structural elements expected to comprise the antizyme inducing motif(s) of small molecules related to agmatine and polyamines.

### BACKGROUND ART

Agmatine is a polyamine produced upon decarboxylation of arginine by arginine decarboxylase (ADC). Thus agmatine is produced by an ADC mediated pathway different from that of a polyamine such as putrescine, which is produced by decarboxylation of ornithine by ornithine decarboxylase (ODC). The polyamines spermidine and spermine are produced by further modification of putrescine. Polyamines are ubiquitous molecules that have been recognized as providing a "buffer" system for the cell by modulating the activities of proteins, RNA, DNA, and lipids in cellular processes such as proliferation, transcription and replication. Moreover, polyamines may also play a direct role in apoptosis. As such, the involvement of polyamines in rapidly proliferating cells, including tumor cells, has been an area of great interest.

Mammals and other organisms have an active polyamine uptake/import and recycling system that complements their polyamine biosynthetic capabilities. Mammalian cells may uptake/import polyamines available in the circulation via gastrointestinal absorption, intestinal flora, or release by other cells. Cellular polyamine levels are also affected by biosynthesis via ODC, which has been shown to be inhibited by isomers of 1,4-dimethylputrescine (Moyano, N. et al., J. Med. Chem., 33:1969-1974 (1990)). Since inhibition of either polyamine uptake/import or biosynthesis results in compensating increases in the other activity, any attempt to lower cellular polyamine levels must target both polyamine transport and biosynthesis (Pegg, A.E. *et al., Int. J. Biochem. Cell. Biol*., 27:425-442 (1995))

Induction of the protein antizyme (AZ) by polyamines is part of the autoregulatory loop controlling cellular polyamine levels. AZ has been shown to bind ODC and inhibit its activity as well as increasing its degradation. Thus AZ induction results in decreased polyamine biosynthesis. AZ has also been shown to inhibit uptake/import activity by polyamine transporters. Recently, ectopically expressed antizyme was shown to have antitumor activity resulting in cell death (Iwata, S. et al., Oncogene, 18(1):165-172, (1999)). Moreover, inducible antizyme expression in nude mice blocked tumor formation.

Agmatine has been recognized as suppressing proliferation by inducing AZ production, resulting in inhibition of both polyamine biosynthesis and uptake/import (Satriano *et al., J. Biol. Chem.,* 273:15313-15316 (1998); WO 98/13037; and Satriano *et al., J. Amer. Soc. Nephrol*., 7:1665 (1996)). Specifically, agmatine induces a +1 translational frameshift of AZ mRNA to result in a full-length protein.

The relationship of polyamines to anticancer therapies has been long recognized. Polyamines affect chromatin structure in eukaryotes and prokaryotes by binding specifically to DNA (Balasundaram, D. *et al*., *Mol. Cell. Biol*. 100:129-140, (1991)) so that condensation occurs when the binding sites on DNA are saturated. Acetylation of polyamines (and histones) lowers their affinity for DNA and is believed to occur in tandem to alter the structure and function of the nucleosome, thus regulating DNA replication and transcription by loosening DNA at the ends of the core particle. The action of novabiocin, an inhibitor of DNA gyrase activity, mimics the effect of polyamine depletion. Polyamines can also modulate DNA binding by certain antibiotics presumably by altering DNA structure (Marton, L. J. *et al*., In: *Inhibition of Polyamine Metabolism,* McCann, P.P. *et al*., Eds., Academic Press, Orlando, Florida, 1987, pp 79-105). Polyamines also affect viral integration into cellular DNA which is also sensitive to chromatin structure (Wallace, H.M. *et al., J. Gen. Virol. 56*:251-258 (1981)). Abnormally low polyamine concentrations cause aberrations in chromatin structure leading to cell death (Quemener, *V. et al., Anticancer Res.* 14:443-448 (1994); Porter, C. W. *et al., Cancer Res.* 53:581-586 (1993)).

Polyamines also have profound effects on RNA, proteins and many cellular functions (Tabor, C. W. *et al., Ann. Rev. Biochem. 53*:749-790 (1984); Ahmed, K. *et al., Adv. Enzyme Regul*. *25*:401-421 (1986); Smith, C. D. *et al., Biochem J.* 256:125-130 (1988)). For example, translation and mRNA splicing are upregulated by polyamines. Polyamines also affect the activity of some protein kinases (Ahmed, *K. et al., Adv. Enzyme Regul.* 25:401-421 (1986)) and lipases, *e*.*g*., phosphatidylinositol phospholipase C (Smith, C.D. *et al., Biochem J.* 256:125-130 (1988)). Polyamines are involved in a one-time modification of a lysine residue of the eIF-5A protein to form hypusine, which is essential for cell viability (Park, M. *H. et al., TIBS* 18:475-479 (1993)). While the exact function of this modified protein is unknown, it exists in all eukaryotes and seems to be associated with cell proliferation. Abnormally high concentrations are associated with apoptosis (Parchment, R. E. *et al., Cancer Res.* 49:6680-6686 (1989)).

The polyamine spermine appears to have an important and specific role in mitochondria. Mitochondria have a specific polyamine transporter (uniporter) that utilizes spermine (Toninello, A. *et al., J. Biol. Chem*. 267:18393-18397 (1992)). This transporter can effectively control the cytosolic Ca⁺² concentration by sequestering these ions in mitochondria (Nicchitta, C. V. *et al., J. Biol. Chem.* 259:12978-12983 (1984)). Several polyamine mimetics also accumulate in the mitochondria to cause loss of DNA and an inability to charge specific tRNAs. In mitochondria, where the secondary structure of tRNAs is minimal, spermine is required for the proper folding of Ile-tRNA (He, Y. *et al., Eur. J. Biochem.* 221: 391-398 (1994)).

It has also been shown that spermine can inhibit the production of proinflammatory cytokines in human mononuclear cells (Zhang, M. et.al. J. Exp. Med 1997, 185 (10), 1759-1768). This suggests that spermine has a counterregulatory mechanism that restrains the immune response in animals. A later report by the same group showed this immune regulatory mechanism of spermine is negated when polyamine transport is inhibited (Zhang, M. et. al. Mol. Med. 1999, 5, 595-605). Taken together, these results suggest that a molecule with the ability to lower the concentration of spermine and other polyamines in immune cells would have immune stimulating effects.

Cellular levels of polyamines are tightly regulated, such that only a small window of variability in polyamine concentration is tolerated. Polyamine concentrations are regulated by modulating their rate of biosynthesis, degradation, uptake/import and efflux. For a review, see Tabor, C. W. *et al*., *Ann. Rev. Biochem.* 53:749-790 (1984). For regulation by controlling import, see Khan, N.A. *et al., Cell Biol. Int'l. Rep.* 15:9-24 (1991). Polyamine transport is highly regulated, subject to feedback inhibition, but remains poorly understood. Cells that up-regulate ODC tend to lose feedback control of the transporter and die when subject to hypotonic stress (Poulin, R. *et al*., *J. Biol. Chem.* 268:4690-4698 (1993)). Recent interest in inhibitors of polyamine transport has been propelled by the finding that polyamine import must also be targeted along with inhibition of polyamine synthesis (Pegg, A. E. *et al., Int. J. Biochem. Cell Biol. 27*:425-442 (1995)). Previously, it was believed that polyamine uptake/import was minimal and that polyamine inhibitors were simply cytostatic, not cytotoxic. However, closer examination of polyamine inhibitors proved that this was not the case. Treatment with polyamine synthesis inhibitors led to upregulated transport so that spermidine levels remained relatively constant. Thus cells compensate for the inhibition of polyamine synthesis by recycling and taking up polyamines from the environment, and in cells subjected to prolonged inhibition of polyamine synthesis, uptake is the only means for survival. Correspondingly, when the intestinal flora of tumor-bearing mice were cleared of polyamine-producing bacteria, inhibitors of polyamine synthesis became cytotoxic (Moulinoux, J.P. et al., J. Urology, 146:1408-1412 (1991) and Quemenor, V. et al., Anticancer Res. 14:443-448 (1994)).

Polyamines can also be salvaged by the two-enzyme recycling system comprising N¹-spermine/spermidine acetyltransferase (SSAT) and polyamine oxidase (PAO). SSAT acetylates the polyamines to produce short-lived intermediates which are either metabolized by PAO or quickly transported out of the cell. PAO oxidizes acetylated spermine to release acetylpropinal and spermidine (or acts on acetylated spermidine to produce putrescine). This reshuffling pathway not only abrogates the requirement for decarboxylated S-adenosylmethionine (dcAdoMet) but also resynthesizes polyamines without production of methylthioadenosine (MTA). It is believed that acetylation is the rate-determining step in the degradative pathway. Several inhibitors upregulate SSAT, which has been recently cloned from rat.

The synthesis of polyamine precursors, putrescine and dcAdoMet, occur respectively through the highly-regulated enzymes ODC and S-adenosylmethionine decarboxylase (AdoMetDC). The polyamine spermidine is synthesized via donation of an aminopropyl group of dcAdoMet to putrescine by the enzyme putrescine aminopropyltransferase (PAPT, also called spermidine synthase) to release one molecule of MTA. Spermidine can be further converted to spermine by donation of another aminopropyl group from dcAdoMet through the action of spermidine aminopropyltransferase (SAPT or spermine synthase) with release of a second molecule of MTA. Both the activities and levels of ODC and AdoMetDC are regulated by hormones, growth factors, polyamine concentration and other factors such as MTA (Janne, *J. et al., Ann. Med.* 23:241-259 (1991); Thomas, T. *et al*., *Canc. Res*. 49:5852-5857 (1989)). For example, MTA can upregulate ODC and down regulate AdoMetDC, thereby increasing the levels of putrescine. The half-lives of these two enzymes are very short, on the order of 30-45 minutes, so their activity can be quickly modulated in accordance with the needs of, and fluctuations in, a dividing cell. Moreover, upregulation of ODC can have oncogenic consequences (Auvinen, M. *et al*., *Nature* 360:355-358 (1992); Holtta, E. *et al., J. Cell Biol*. 122:903-914 (1993)). This oncogenic transformation was inhibited by the ODC inhibitor α-difluoromethylornithine (DPMO). In a colon cancer model, rearrangements of the ODC gene were related to the grade of malignancy (Pascale, R. M. *et al*., *Carcinogenesis*. 14:1077-1080 (1993)).

### DISCLOSURE OF THE INVENTION

The present invention is directed to agmatine and polyamine analogs and their use as drugs, as well as agricultural or environmentally useful agents. As drugs, the agmatine and polyamine analogs decrease cellular polyamine levels and can be used to treat disorders of undesired cell proliferation, including cancer, viral infections and bacterial infections. The analogs may be utilized alone or in combination with other agents, particularly other inhibitors of polyamine synthesis or transport, but including other inhibitors of cell proliferation. Without being bound by theory, the agmatine and polyamine analogs of the invention may decrease polyamine levels by increasing expression of full length antizyme (AZ) inducing a +1 translational frameshift of AZ mRNA to permit expression of the full-length protein.

which in turn down regulates both the production of polyamines by ornithine decarboxylase (ODC) and the transport of polyamines by its corresponding transporter (PAT). The invention further defines structural elements/motifs within these analogs that appear key to their induction of antizyme as determined by assays. Because polyamines are absolutely essential for DNA replication and are essential to cellular homeostasis, there is an interest in preventing cell proliferation by lowering intracellular polyamine levels. Sufficiently low polyamine levels can lead to cell death. Thus any agent able to lower polyamine levels, particularly by inhibiting both polyamine biosynthesis and uptake/import, offers the opportunity to target a variety of disease or undesirable conditions related to cell proliferation, including cancer.

The analogs of the invention are not necessarily metabolized like agmatine, which is metabolized by diamine oxidase to guanidinobuteraldehyde or by agmatine-specific agmatinase to putrescine, or like naturally occurring polyamines. As such, the analogs of the invention have the advantage of not being readily metabolized to regenerate polyamines. Administration of agmatine or metabolizable polyamines, which are subject to conversion to putrescine and other polyamines, would be expected to defeat the goal of decreasing polyamine levels . Thus one aspect of the invention is the production and use of agmatine and polyamine analogs that are not metabolized to putrescine or any other naturally occurring polyamine metabolite. On the other hand, the normal metabolism of agmatine and other polyamines may be utilized to result in the intracellular production of an agmatine or polyamine analog. Thus another aspect of the invention is the production and use of prodrug forms of the disclosed analogs. The invention additionally encompasses the stabilization of agmatine and polyamine analogs by modifying them to resist enzymatic degradation as an alternative. Such modifications include substitution of primary amine groups with alkyl groups.

Moreover, while it is unknown whether agmatine upregulates SSAT or PAO, involved in polyamine salvage as described above, or AdoMetDC, involved in polyamine synthesis as described above, the fact that these enzymes may be regulated by agmatine renders it possible that the agmatine and polyamine analogs of the invention will modulate these enzymes as well. This expectation is based on the observation that if agmatine, and hence putrescine, levels are high, cells would tend to upregulate enzymes which lower polyamine levels while inhibiting enzymes that contribute to polyamine synthesis.

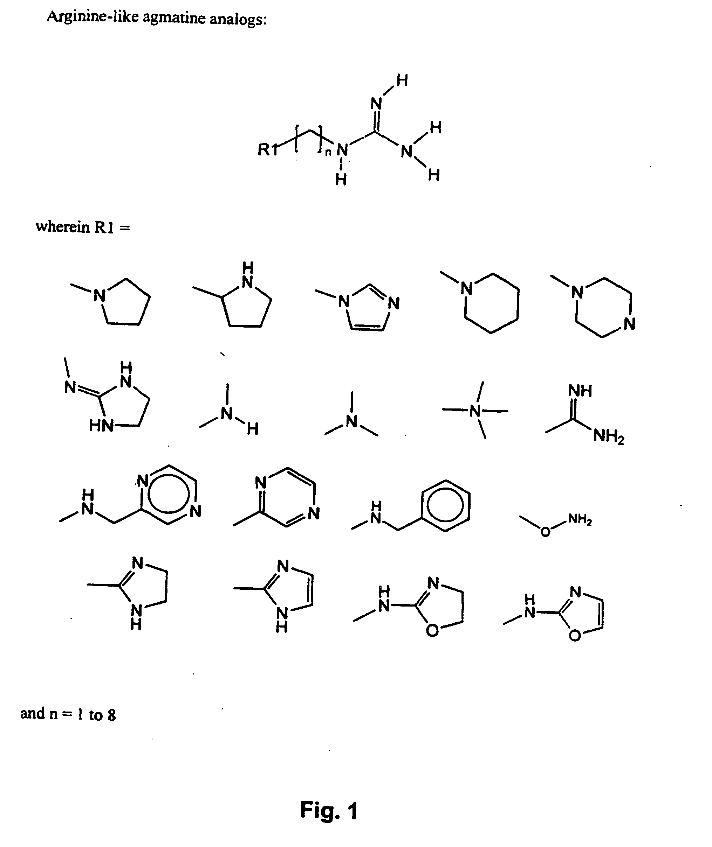

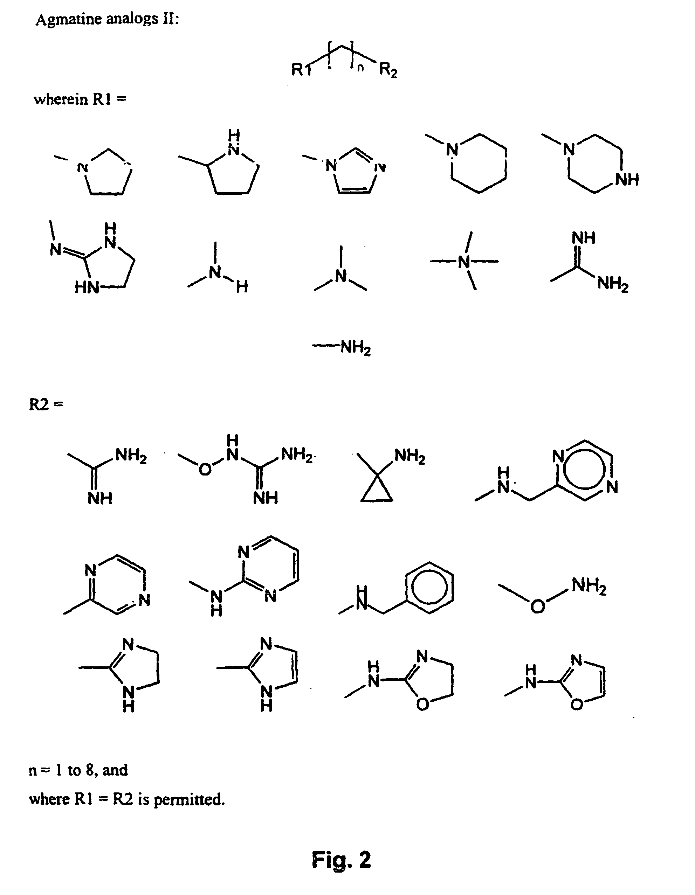

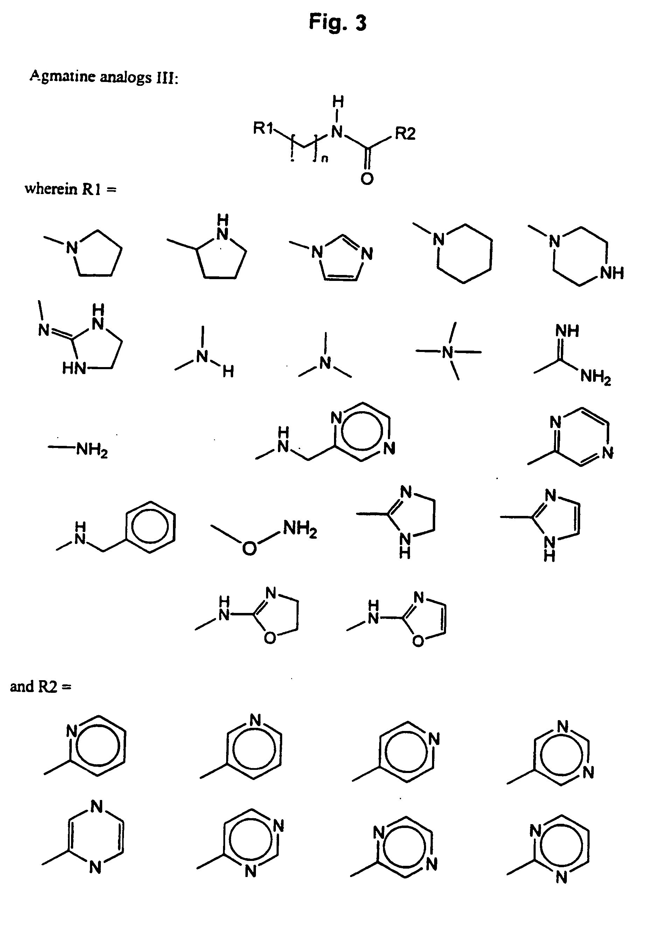

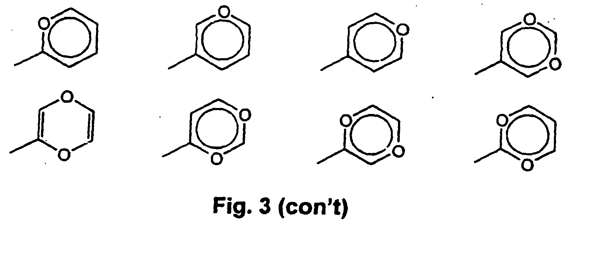

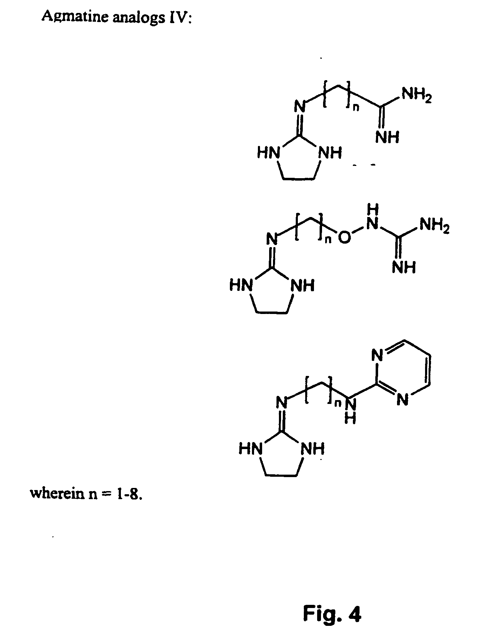

One series of agmatine analogs of the present invention are those as set forth in Figures 1-4 and 6, described below. These analogs include those substituted at one or more positions. Disubstituted agmatines are preferably substituted at the two terminal nitrogens, but may be alternatively or additionally substituted at internal nitrogen and/or internal carbon atoms. An additional series of agmatine analogs are related to N¹, N⁴-dibenzylputrescine, which has activity against tumor cells in culture (Aizencang, G. *et al., Cellular and Molecular Biology,* 44(4):615-625 (1998)). The report describes experiments that suggest its mechanism might be related to antizyme induction. Treatment of cells with this compound (or several related analogs) effectively lowered all polyamine levels after 72 hours of incubation. N¹, N⁴-dibenzylputrescine and several very closely related analogs are also disclosed in U.S. Pat. 5,677,350. The present inventors propose that dibenzyl diaminoalkane analogs and derivatives would be good compounds for anticancer agents possibly via the mechanism of antizyme induction. Since a biological binding site for agmatine may be the ribosomes, tRNA(s) or rRNA(s) involved in frame-shift translation of the antizyme message, and the alkylated putrescine analogs of Aizencang *et al*. apparently can also act there, it is clear that optimization of the binding of the instant agmatine analogs will occur around positions corresponding to the benzyl lead of the Aizencang *et al*. compounds. The present inventors expect that substituents on these rings can increase binding affinity for ribosomes, tRNA or rRNA by adding more sites for interaction.

The inventors have also recognized that agmatine is structurally related to a decarboxylated product of L-canavanine, guanidinooxypropylamine (GOPA). L-canavanine, an arginine amino-acid analog, is a natural plant product (see Miersch, J. *et al.,* In: *Guanidino Compounds in Biology and Medicine:* 2, De Deyn, P.P. et al., Eds., John Libbey & Co., London, England, 1997, pp.401-405), and has been shown in the literature to be incorporated into proteins in place of arginine. Due to the greatly reduced basicity of the guanidino functionality, such proteins have altered properties and stabilities. L-canavanine has also been shown to have antitumor activity in rats, is commercially available from Sigma Chemical Company (St. Louis, Missouri), and is modified to GOPA by reaction with *E. coli* ADC (Hamana, K. *et al*., *Biochem. Biophys. Res. Comm.,* 129(1):46-51 (1985)), also available from Sigma Chemical Company. Thus agmatine analogs that are structurally similar or related to GOPA are yet another embodiment of the invention.

Additionally, the agmatine and polyamine analogs of the invention are designed to enter cells by pathways other than those of active polyamine transport regulated by AZ. For example, bis(benzyl)polyamine analogues are substrates for a mammalian cell-transport system which is distinct from the polyamine transport system (Beyers, T.L. et al., Biochem. J. 269:35-40 (1990)). Additionally, agmatine and polyamines have been shown not to share the same transporter (Sastre, M. *et al., J. Neurochem.,* 69:2421-2426 (1997)). For example, agmatine uptake into rat synaptosomes is not inhibited by the polyamines putrescine, spermidine, or spermine at a concentration of 1 mM. Instead, agmatine uptake may occur through a Ca²⁺ subtype transporter. Thus, an additional embodiment of the invention are analogs that are not imported into cells by the polyamine transporters.

The invention also includes the results of an exploration of the structural requirements for antizyme induction by polyamine analogs. Specifically, the structural and charge profiles of analogs based on three naturally occurring polyamines, spermine, spermidine, and putrescine have been studied relative to the ability to induce antizyme. It has been previously observed that amongst spermine, spermidine, and putrescine, the ability to induce full-length antizyme production is greatest with spermine and lowest with putrescine.

In addition to the differences in size and complexity found in analogs derived from these three polyamines, there is a difference in the number of positive ammonium cationic charges present in the molecule at physiological pH. For example, at pH 7.2, the number of positive charges in the three polyamines, and thus the corresponding analogs, is spermine (+4) > spermidine (+3) > putrescine (+2).

Beyond the involvement of size and charge, however, the invention includes analogs having specific separations between the charge centers as well as the intervening structure(s) between charge centers. Additionally, analogs of the invention differ from naturally occuring polyamines by having specific chemical moieties as substituents on the polyamine backbone.

A preferred embodiment is a highly specific agmatine or polyamine analog which decreases cellular polyamine levels, and accordingly cell proliferation, and has pharmaceutical utility as an anti-cancer chemotherapeutic. Such analogs may act by inducing AZ.

An additional embodiment of the invention are agmatine and polyamine analogs that deplete cellular polyamine levels to regulate an immune response. A preferred embodiment of the invention is an agmatine or polyamine analog which lowers the concentration of spermine and other polyamines in immune cells to produce immune stimulating effects. Such analogs would be particular advantage in uses relating to bacterial or viral infections, immuno-compromized patients and cancer treatments.

The invention also provides a pharmaceutical composition useful for treating a disease or condition in which decreases in cellular polyamine levels is desirable, comprising an agmatine or polyamine analog as described herein and a pharmaceutically acceptable excipient.

Additionally, the invention provides a method for treating a disease or a condition in a subject associated with undesired cell proliferation which is treatable by decreases in cellular polyamine levels, inhibition of polyamine biosynthesis, and/or inhibition of polyamine uptake, comprising administering to said subject a pharmaceutical composition comprising an agmatine or polyamine analog as described herein. The disease is preferably cancer.

The invention further provides for combination compositions and methods comprising the above analogs with other anti-proliferative agents. Because polyamines affect chromatin structure (Knuutila, S. *et al., Exp. Cell. Res.* 145:222-226 (1983)), anti-tumor agents such as topoisomerase II inhibitors or other intercalating agents are more effective against proliferating cells in combination with the instant agmatine and polyamine analogs. It may be likely that such combinations will have synergistic effects in cytoxicity which are greater than the toxicity of each individual agent. Thus such combination drug therapies would be expected to require lower doses of the instant analogs and the other anti-proliferative agent(s).

Other aspects of the invention include methods of assaying analogs for the ability to induce AZ and methods of assaying for polyamine transport. Additional embodiments of the invention include the identification of proteins or nucleic acids that bind to agmatine and polyamine analogs, the histochemical or cytochemical localization of cellular factors involved in antizyme induction, as well as structural elements/motifs of small molecules that participate in AZ induction.

### BRIEF DESCRIPTION OF THE DRAWINGS

Figure 1 shows the structures of arginine-like agmatine analogs of the invention.
Figure 2 shows additional agmatine analogs comprising variable amine groups.
Figure 3 shows additional agmatine analogs comprising a heterocyclic ring.
Figure 4 shows additional agmatine analogs encompassed by the invention.

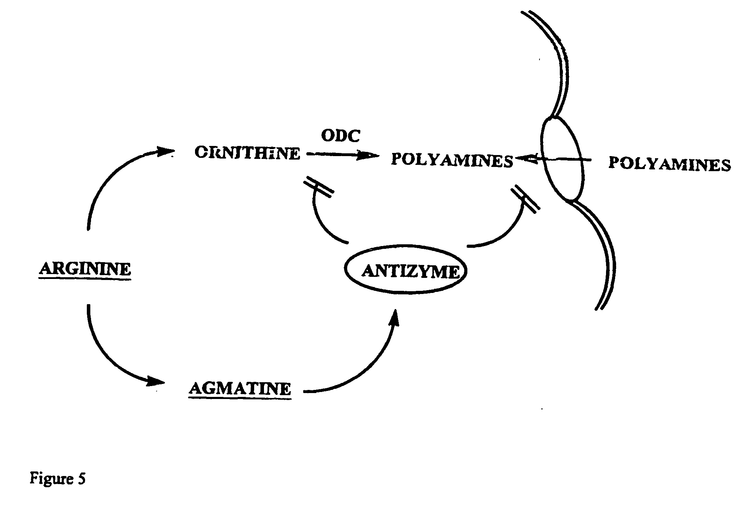

Figure 5 is a schematic illustrating polyamine regulation by agmatine via antizyme.
Figure 6 shows a agmatine analogs similar to amidine, acetylimide and comprising a guanidinoxy motif.
Figure 7 is a schematic proposing the production of GOPA by ADC *in vivo*.
Figure 8 shows the structures of agmatine and various related compounds.

### MODES FOR CARRYING OUT THE INVENTION

By agmatine and polyamine "analogs", the invention encompasses compounds related in structure to that of agmatine and polyamines as well as to compounds derived from agmatine and polyamines, or "derivatives" by modification of one or more atoms or bonds normally present in agmatine and polyamines. Moreover, the "analogs" of the invention are those able to lower cellular polyamine levels when administered to cells. Preferably, the "analogs" lower polyamine levels by the induction of antizyme. More preferably, the "analogs" are not metabolizable to structures that contribute back to the cellular polyamine pool. Furthermore, the "analogs" of the invention are preferably imported into cells by mechanisms independent of polyamine transport.

The structural depictions of agmatine and polyamine "analogs" of the invention are usually as the free base form. However, the "analogs" of the invention include the corresponding protonated forms of the structural depictions. Moreover, the "analogs" include the corresponding salt forms of the protonated species.

The analogs of the invention are capable of forming positive charges at physiological pH, which is defined as ranging from 6.0 to 8.0. More preferrably, the range is 6.5 to 7.5. Most preferrably the pH is defined as 7.0 or 7.2. It will be appreciated by one skilled in the art that all subrange pH values within 6.0 to 8.0 and 6.5 to 7.5, as well as individual pH values, such as 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9 and 8.0 are within the scope of the product analogs and methods of the invention.

It is known that primary and secondary amine groups are protonated at physiological pH. A "rule of 5" has also been set forth in the art to estimate solubility and permeability of drugs as well as predict their oral uptake and tissue distribution (Lipinski, C.A. et al., Adv. Drug Deliv. Rev., 23:3-25 (1997)). Given this knowledge, the particular form of the agmatine and polyamine "analog" will determine in part the method of its administration. For example, agmatine and polyamine analogs outside the "rule of 5" may be administered by more direct methods, such as intravenously.

Another general property of the "analogs" of the invention with particular significance for the practice of the invention is a relative absence of toxicity to cells that are not rapidly proliferating. Whether the "analogs" of the invention are able to lower polyamine levels and hence inhibit cell proliferation can be easily and quickly determined by the assays provided below.

The present inventors have designed novel agmatine and polyamine analogs for therapeutic uses and have devised tests using such compounds as probes for measuring antizyme (AZ) induction. Such analogs lower cellular polyamine levels and are useful as drugs in a number of diseases, particularly cancer. They may also have the ability to induce AZ which lowers cellular polyamine levels by inhibiting both polyamine biosynthesis and transport. They can also be used as a component of novel drug combinations with, for example, a polyamine biosynthesis inhibitor such as α-difluoromethylornithine (DFMO), which inhibits ornithine decarboxylase, or with other agents, including antitumor agents or other drugs used to treat disorders of undesired cell proliferation. The analogs of the present invention are also useful in other diseases or conditions in which polyamines play a role as described above, and have agricultural and environmental uses. Preferably, the analogs enter cells by pathways other than those of regular polyamine transport.

Spermine analogs of the invention generally contain four amine groups capable of forming four positive charges at physiological pH, wherein the first and second amine groups, and the third and fourth amine groups, are separated by the distance of four C-C and/or C-N bonds and said second and third amine groups are separated by the distance of five C-C and/or C-N bonds or more. Preferably said second and third amine groups are separated by -CH₂-Group-CH₂- wherein said Group is a C₂₋₁₀, straight or branched, alkyl, alkenyl, alkynyl, alkoxy, or aliphatic; C₃₋₁₀ alicyclic; single or multi-ring aromatic or aryl; aryl-substituted alkyl, alkenyl or alkynyl; single or multi-ring aryl substituted aliphatic; aliphatic-substituted single or multi-ring aromatic; alkyl-, alkenyl-, or alkynyl-substituted aryl; a single or multi-ring heterocyclic; a single or multi-ring heterocyclic-substituted aliphatic; an aliphatic-substituted aromatic; heterocyclic-substituted alkyl, alkenyl or alkynyl; or alkyl-, alkenyl-, or alkynyl-substituted heterocyclic.

The Group of more preferred spermine analogs is a single or multi-ring aromatic or aryl; aliphatic-substituted single or multi-ring aromatic; alkyl-, alkenyl-, or alkynyl-substituted aryl; a single or multi-ring heterocyclic; an aliphatic-substituted aromatic; or alkyl-, alkenyl-, or alkynyl-substituted heterocyclic. The Group of especially preferred spermine analogs is any one of the following: 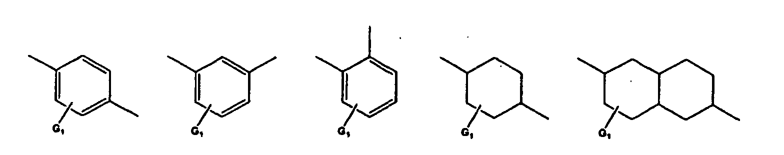 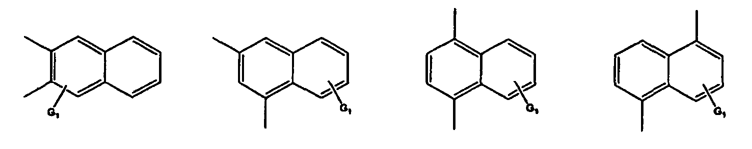 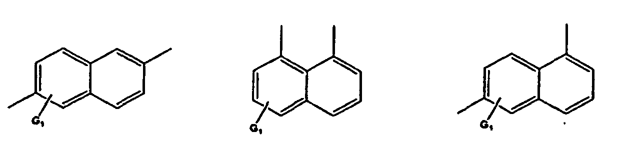 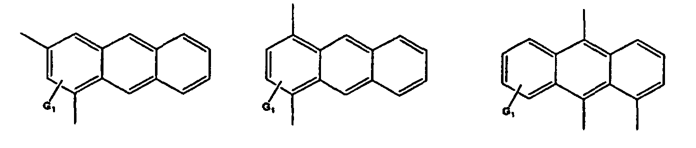 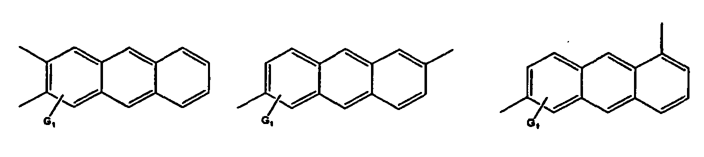 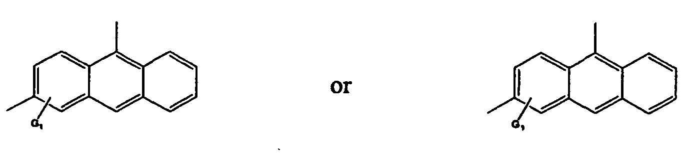 wherein G₁ is -H; a C₂₋₁₀, straight or branched, alkyl, alkenyl, alkynyl, alkoxy, or aliphatic; C₃₋₁₀ alicyclic; single or multi-ring aromatic or aryl; aryl-substituted alkyl, alkenyl or alkynyl; single or multi-ring aryl substituted aliphatic; aliphatic-substituted single or multi-ring aromatic; alkyl-, alkenyl-, or alkynyl-substituted aryl; a single or multi-ring heterocyclic; a single or multi-ring heterocyclic-substituted aliphatic; an aliphatic-substituted aromatic; heterocyclic-substituted alkyl, alkenyl or alkynyl; alkyl-, alkenyl-, or alkynyl-substituted heterocyclic; or combinations thereof.

In particularly preferred spermine analogs, G₁ is -H, -CH₃, -CH₂CH₃, -CH₂Ph, -NH₂, -NHCOCH₃, -N₃, -CN, -F, -CL, -BR, -I, -CF₃, -OCH₃, -OCH₂Ph, -COCH₃, -COOH, -COOCH₃, -COOCH₂CH₃, -COOCH₂Ph, -OCH₂CH₂O-, -C(NH₂)=NH, or combinations thereof; where "Ph" is phenyl.

Spermine analogs of the invention may also contain a substituent on one or more of its amine groups or on one or more of the carbon atoms adjacent thereto. Preferably, the adjacent carbon atoms are the terminal carbon atoms in spermine. The substituent of such analogs is preferably a C₁₋₁₀, straight or branched, alkyl, alkenyl, alkynyl, alkoxy, or aliphatic; C₃₋₁₀ alicyclic; single or multi-ring aromatic or aryl; aryl-substituted alkyl, alkenyl or alkynyl; single or multi-ring aryl substituted aliphatic; aliphatic-substituted single or multi-ring aromatic; alkyl-, alkenyl-, or alkynyl-substituted aryl; a single or multi-ring heterocyclic; a single or multi-ring heterocyclic-substituted aliphatic; an aliphatic-substituted aromatic; heterocyclic-substituted alkyl, alkenyl or alkynyl; or alkyl-, alkenyl-, or alkynyl-substituted heterocyclic. Most preferably, the substituent is methyl, ethyl, phenyl, or CH₂Ph, where "Ph" is phenyl.

Spermidine analogs of the invention contain three amine groups capable of forming three positive charges at physiological pH, wherein the first and second amine groups are separated by the distance of five C-C and/or C-N bonds and said second and third amine groups are separated by the distance of four C-C and/or C-N bonds. Preferably, the analogs further contain a substituent on one or more of said amine groups or on one or more of the carbon atoms adjacent thereto. Most preferred is for the one or more adjacent carbon atoms to be one or both of the terminal carbon atoms in spermine.

Also preferred are spermidine analogs containing a carboxylic amide or sulfonamide amide on the N¹-position of spermidine. More preferably, the carboxylic amide is an alkyl or aryl carboxylic substituent comprising a C₂₋₁₀, straight or branched, alkyl, alkenyl, alkynyl, alkoxy, or aliphatic; C₃₋₁₀ alicyclic; single or multi-ring aromatic or aryl; aryl-substituted alkyl, alkenyl or alkynyl; single or multi-ring aryl substituted aliphatic; aliphatic-substituted single or multi-ring aromatic; alkyl-, alkenyl-, or alkynyl-substituted aryl; a single or multi-ring heterocyclic; a single or multi-ring heterocyclic-substituted aliphatic; an aliphatic-substituted aromatic; heterocyclic-substituted alkyl, alkenyl or alkynyl; or alkyl-, alkenyl-, or alkynyl-substituted heterocyclic. Also preferred are where the sulfonamide amide is an alkyl or aryl sulfonamide substituent comprising a C₂₋₁₀, straight or branched, alkyl, alkenyl, alkynyl, alkoxy, or aliphatic; C₃₋₁₀ alicyclic; single or multi-ring aromatic or aryl; aryl-substituted alkyl, alkenyl or alkynyl; single or multi-ring aryl substituted aliphatic; aliphatic-substituted single or multi-ring aromatic; alkyl-, alkenyl-, or alkynyl-substituted aryl; a single or multi-ring heterocyclic; a single or multi-ring heterocyclic-substituted aliphatic; an aliphatic-substituted aromatic; heterocyclic-substituted alkyl, alkenyl or alkynyl; or alkyl-, alkenyl-, or alkynyl-substituted heterocyclic.

Most preferred alkyl containing substituents further contains a halide, such as fluoro, chloro, bromo, or iodo) or said alkoxy moiety is methoxy, ethoxy, or substituted or unsubstituted benzyloxy.

Examples of such analogs include the following:

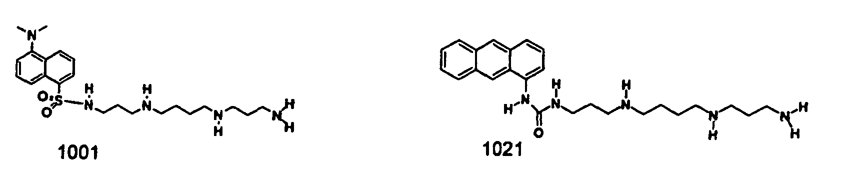

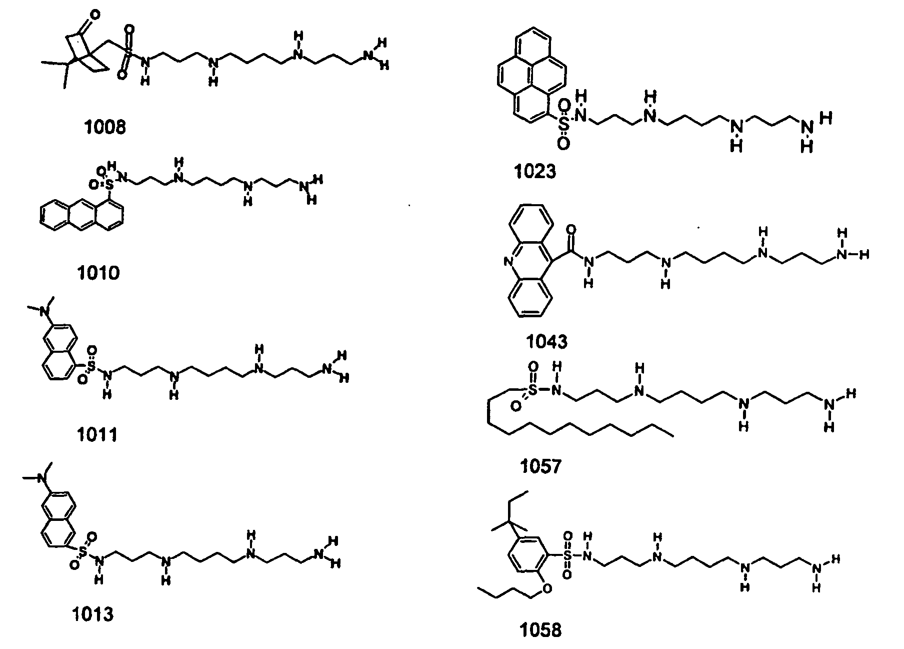

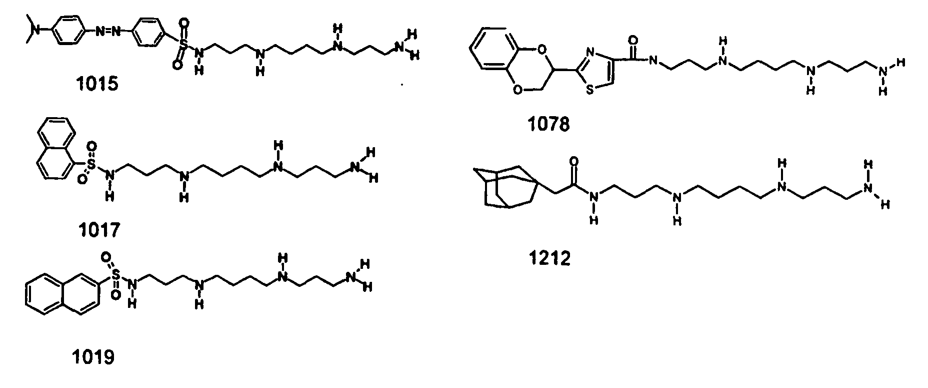

Putrescine analogs of the invention contain two amine groups capable of forming two positive charges at physiological pH, separated by the distance of five C-C, C-O, N-O, and/or C-N bonds or more. Preferably, the analogs also contain one or more substituents on one or more of said amine groups or on one or more adjacent carbon atoms. These substituents are preferably a C₁₋₁₀, straight or branched, alkyl, alkenyl, alkynyl, alkoxy, or aliphatic; C₃₋₁₀ alicyclic; single or multi-ring aromatic or aryl; aryl-substituted alkyl, alkenyl or alkynyl; single or multi-ring aryl substituted aliphatic; aliphatic-substituted single or multi-ring aromatic; alkyl-, alkenyl-, or alkynyl-substituted aryl; a single or multi-ring heterocyclic; a single or multi-ring heterocyclic-substituted aliphatic; an aliphatic-substituted aromatic; heterocyclic-substituted alkyl, alkenyl or alkynyl; or alkyl-, alkenyl-, or alkynyl-substituted heterocyclic. More preferably, the substituents are -H, -CH₃, -CH₂CH₃, or -CH₂CH₂-.

Especially preferred analogs are 1,4-diaminobutane with monomethyl or dimethyl substitutents in the 1, 2, 3 or 4 position; 1,4-, 1,3-, or 1,2- dimethyl-diaminobutane; bis-1,4-cyclopropyl-1,4-diaminobutane; and 1,1,4,4-tetramethyldiaminobutane. Even more preferred are analogs having the following 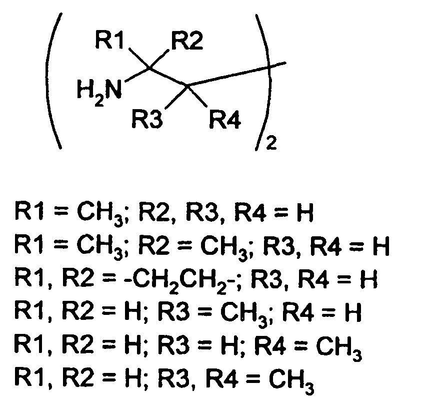 structure:

Other analogs of the invention include dibenzylputrescine analogs, including *N*¹, N⁴-Bis methylene or ethylenearyl-1,X-Diaminoalkane or arene-diaminoalkane. Additional analogs of the invention have the following structure:

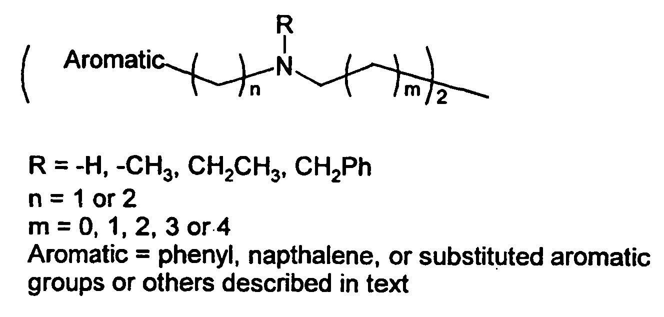

Moreover, 1-Guanidinoxy 3-aminopropane derivatives, such as 1, 2, or 3-monomethyl GOPA and 3,3-dimethyl-GOPA are analogs of the invention as well. Additional analogs of the invention have the following structure:

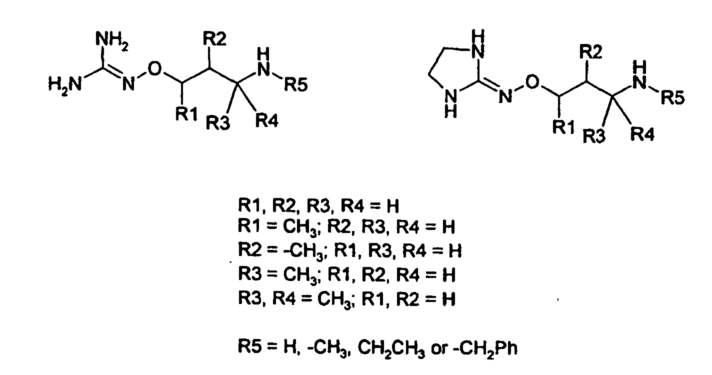

The present invention also provides a composition which comprises an analog that decreases cellular polyamine levels and/or induces AZ. These analogs are encompassed by the above description as well as the formulas set forth in Figures 1-4 and 6.

Representative spermine analogs of the invention, with a +4 charge from amine groups at pH 7.2, are set forth in the following Table:

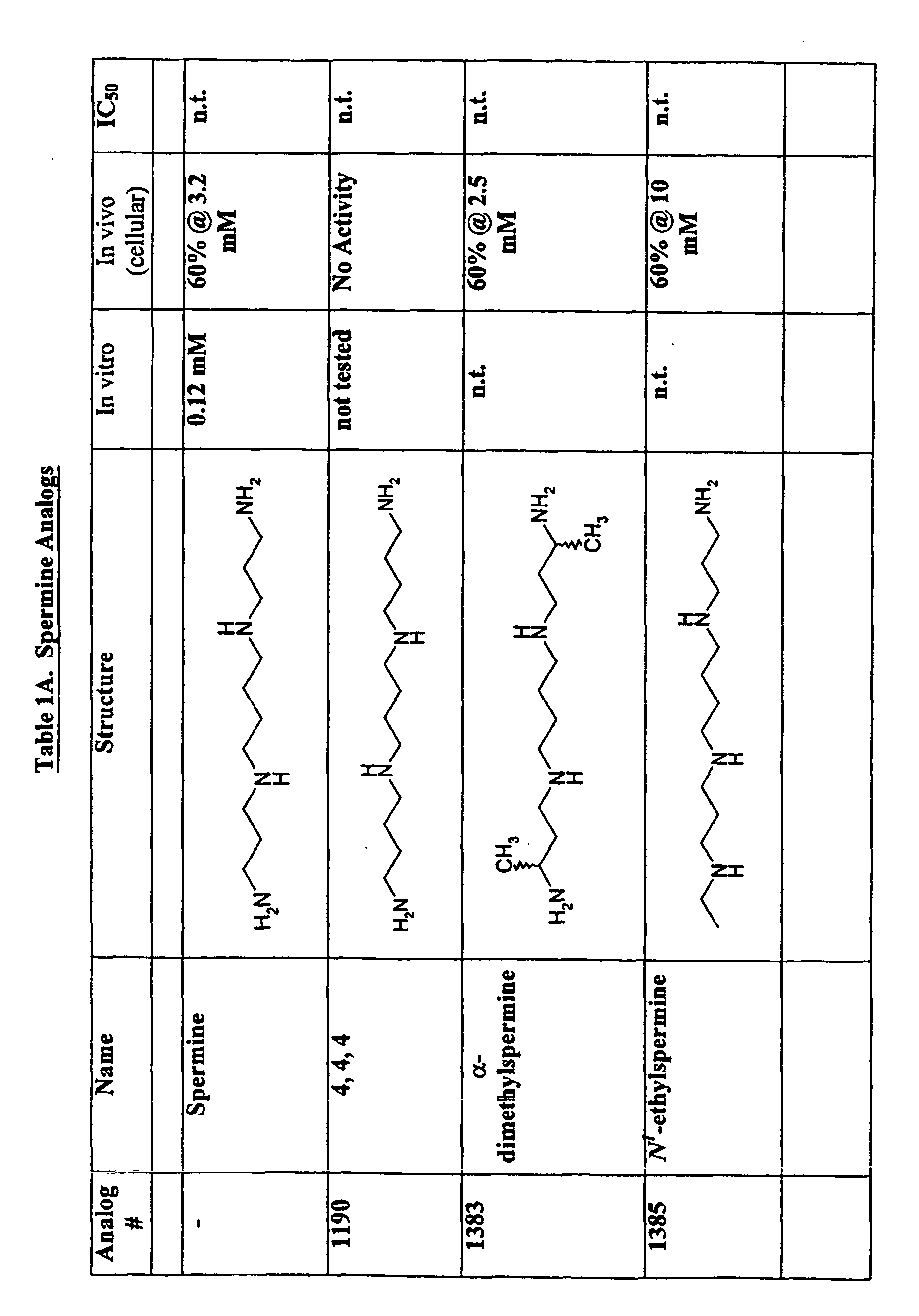

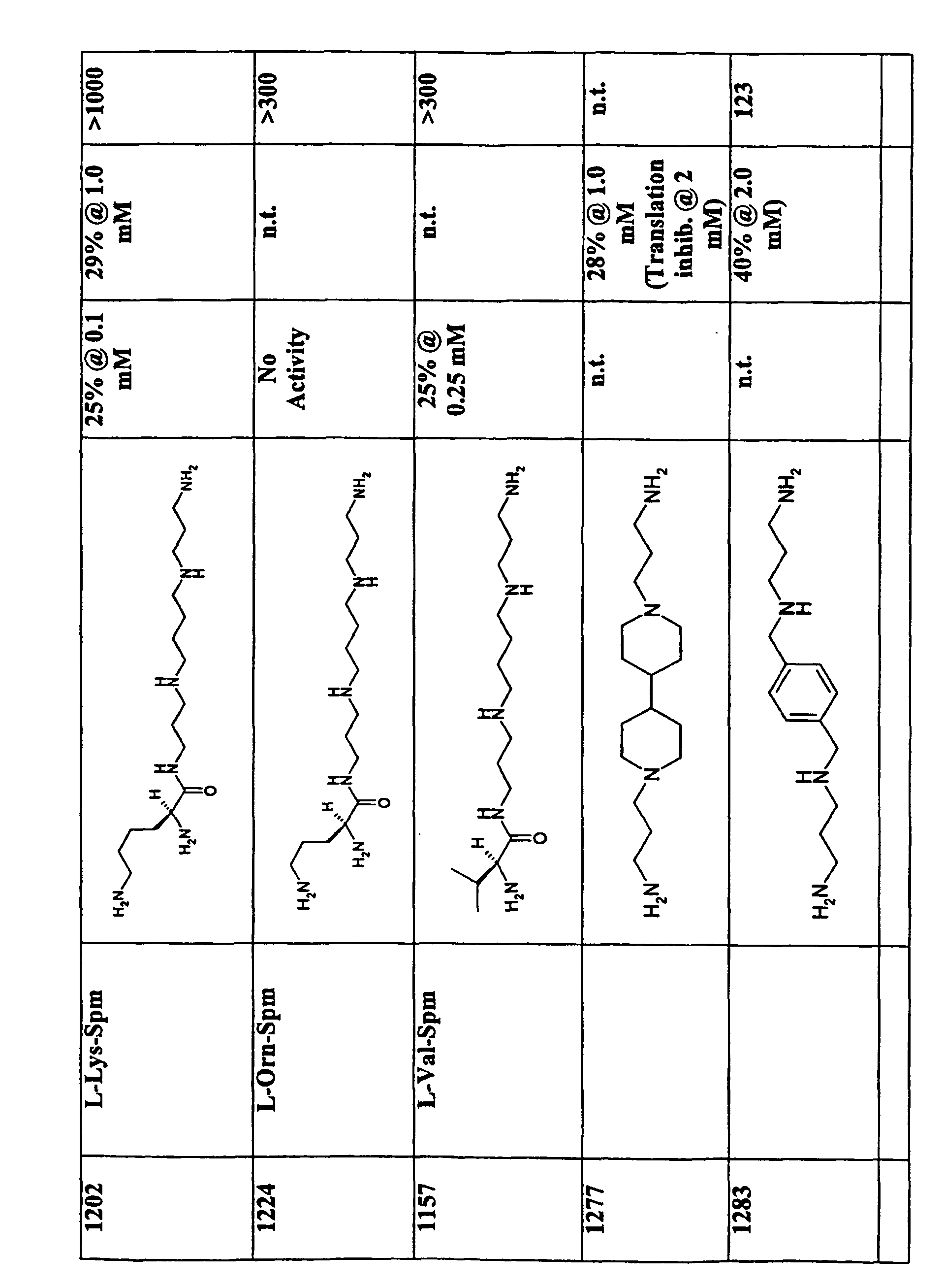

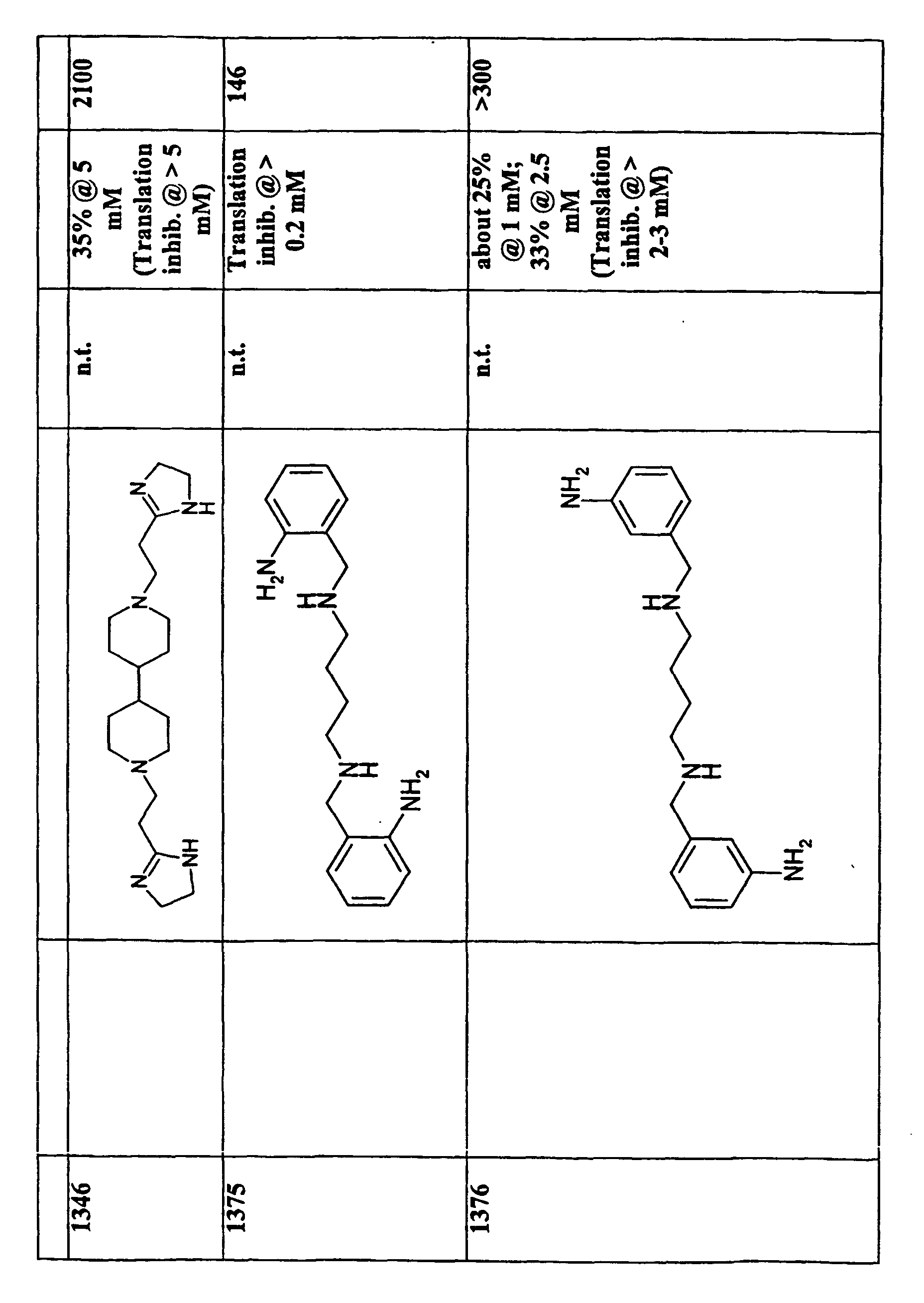

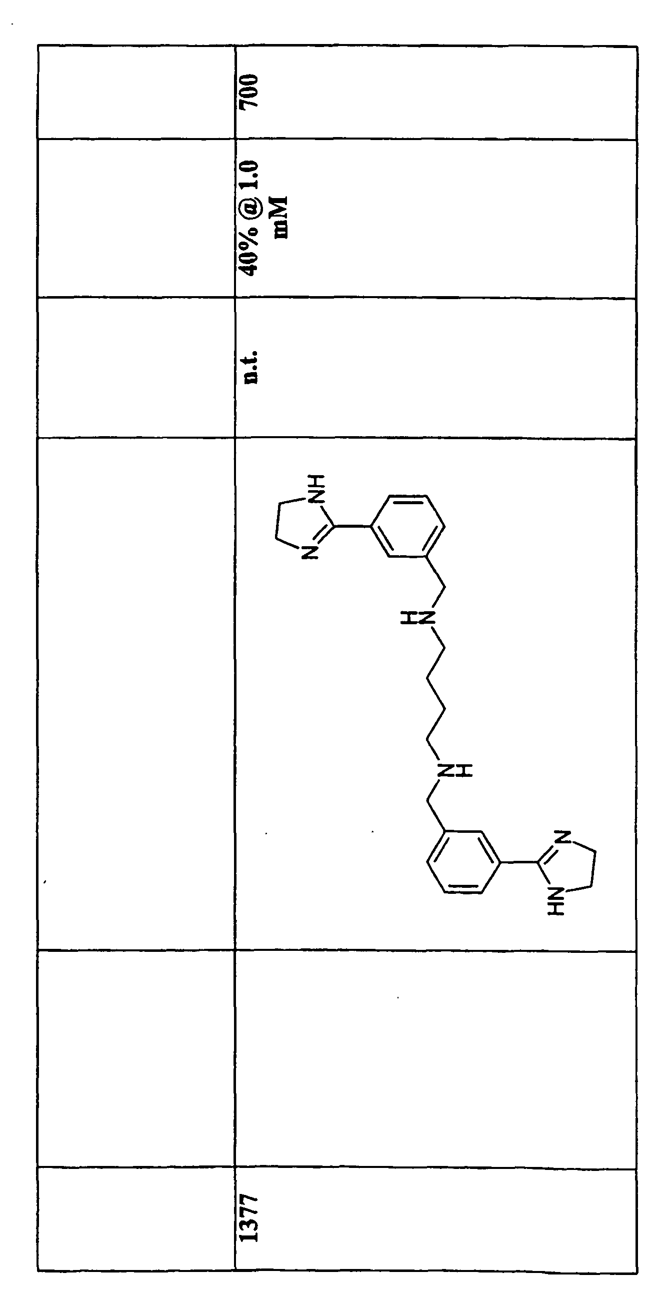

The IC50 values in the above Table as well as Tables 1B and 1C below were determined with the breast carcinoma cell line, MDA-MB-231.

Tests on ten of the analogs listed in Table 1A in an *in vivo* cellular antizyme induction assay revealed eight positive results. Specifically, analogs 1383, 1385, 1202, 1277, 1283, 1346, 1376 and 1377 tested positive *in vivo* while analogs 1190 and 1375 tested negative. Surprisingly, analog 1375 was found to significantly inhibit protein translation in the assay before any antizyme induction could be detected while analogs 1277, 1346 and 1376 inhibited translation at concentrations higher than those needed to induce antizyme. These analogs may be useful as inhibitors of cellular translation of RNA transcripts.

The importance of the distance between positive charge centers is demonstrated by a comparison of analog 1190 and spermine, which induced antizyme in both the *in vitro* and *in vivo* assays. Analog 1190, which replaces both aminopropyl groups in spermine with one-carbon atom extended aminobutyl groups, showed no ability to induce antizyme *in vivo.* Another spermine analog, 1383 or α-dimethylspermine, has two methyl groups on the terminal carbon atoms of the aminopropyl groups of spermine and demonstrated an ability to induce antizyme in the *in vivo* cellular assay to the same extent as spermine. In addition to the same spacing between positive charge centers as spermine, this molecule has been shown by others to have a dramatically reduced metabolism rate in tumor cells.

Analog 1385, or *N*^{*1*}-ethylspermine, is a derivative of spermine with monoalkylation of a terminal nitrogen with ethyl. It also maintains the spacing between positive charge centers and was only slightly less able to induce antizyme *in vivo* when compared to spermine.

Analogs 1202, 1224 and 1157 are conjugates of amino acids and spermine which demonstrate the importance of the structure of substituents in a polyamine analog. The L-Lys-spermine conjugate, analog 1202, showed a diminished ability to induce antizyme either *in vitro* or *in vivo* in comparison to spermine, while a loss of induction was seen *in vitro* with analog 1224, which has one less methylene carbon atom compared to analog 1202. The third analog, 1157 or L-Val-spermine, retained the ability to induce antizyme *in vitro*. The observation with analog 1224 may be analogous to a similar situation with cadaverine (see Table 1C below) where a change in the spacing between the two positive charge centers in putrescine resulted in a dramatic change in *in vitro* antizyme induction but little difference *in vivo.*

The last six analogs in Table 1A contain modifications of the spermine backbone. Substitution of the internal diaminobutane carbon chain of spermine with a more conformationally rigid bicyclic ring system results in analog 1277, which demonstrated a diminished ability to induce antizyme *in vivo.* An increase in induction activity is observed with analog 1283, where the diaminobutane portion of spermine is replace by a bis-1,4-(aminomethyl)benzene substitution. An additional analog of compound 1283, shown below, contains methyl groups on the terminal carbon atoms of the aminopropyl groups which diminishes its ability to be metabolized *in vivo.*

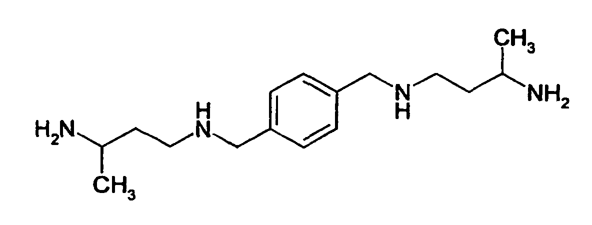

Replacement of the terminal amino groups of analog 1277 with dihydroimidazolium groups results in compound 1346, with slightly better ability to induce antizyme.

Representative spermidine analogs of the invention, with a +3 charge from amine groups at pH 7.2, are set forth in the following Table

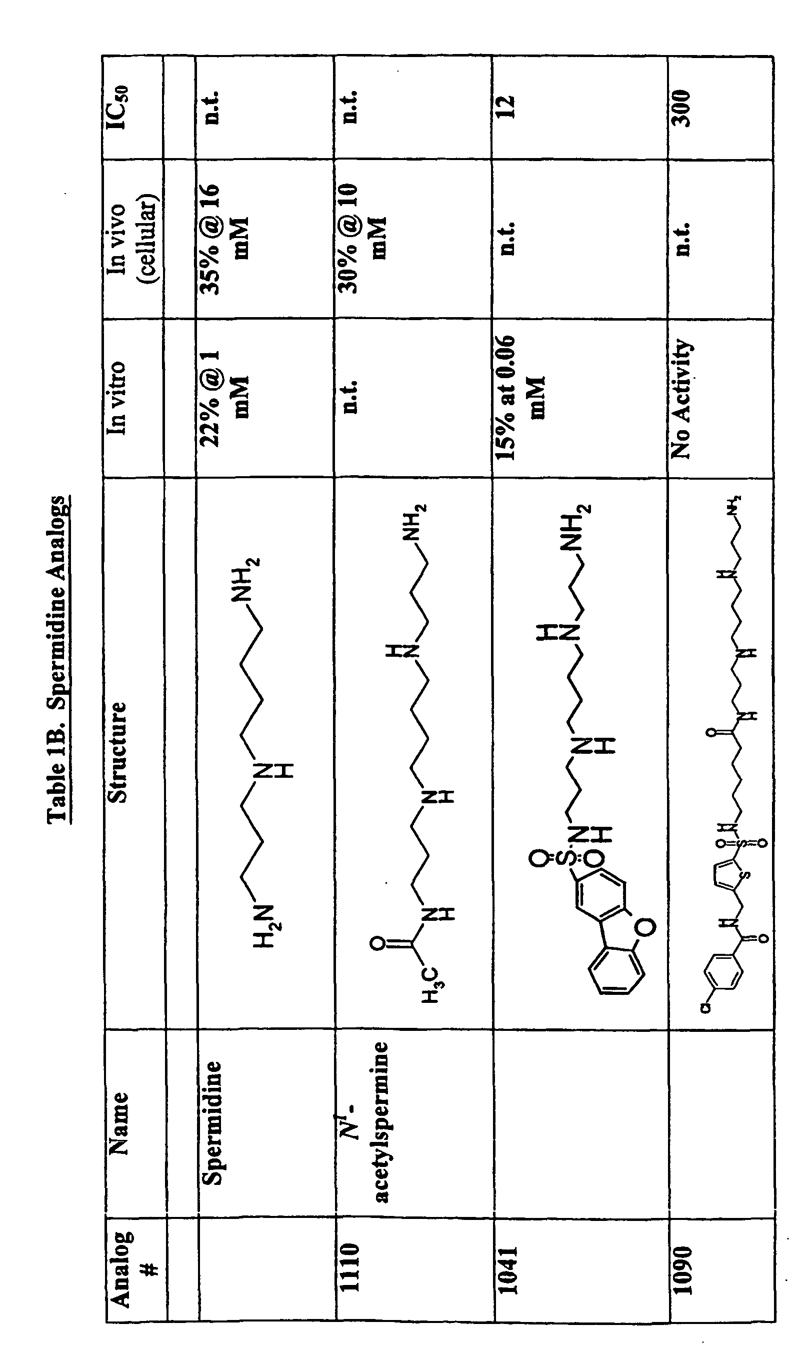

Of the analogs in Table 1B, compound 1110 demonstrated antizyme inducing activity in an in *vivo* cellular assay while 1041, which has yet to be tested *in vivo,* demonstrated *in vitro* induction activity. Analog 1110 (*N*^{*1*}-acetylspermine) is the product of adding an acetyl group to spermine to reduce the molecule's number of positive charges from +4 to +3. This molecule, (ORI 1110), showed a diminished ability to induce the expression of active antizyme in comparison with spermidine although they have an equal number of charges.

In contrast, modification of spermine with a large aromatic group, through a sulfonamide link to produce analog 1041, gives a molecule with a dramatic increase in *in vitro* induction activity in comparison with spermidine. This result suggests an aromatic binding region, or pocket, in the target for antizyme induction.

The next analog in this series, compound 1090, suggests that the binding region has specificity for certain structural groups. Analog 1090 contains a 6-aminohexanoic acid linker between the aromatic portion and spermine. While analog 1090 demonstrated no ability to induce antizyme in the *in vitro* assay, it too may be analogous to the situation observed with cadaverine (see Table 1C below) where a change in the spacing between the two positive charge centers in putrescine resulted in a dramatic change in *in vitro* antizyme induction but little difference *in vivo.*

Representative putrescine analogs, with a +2 charge from amine groups at pH 7.2, are as set forth in the following Table:

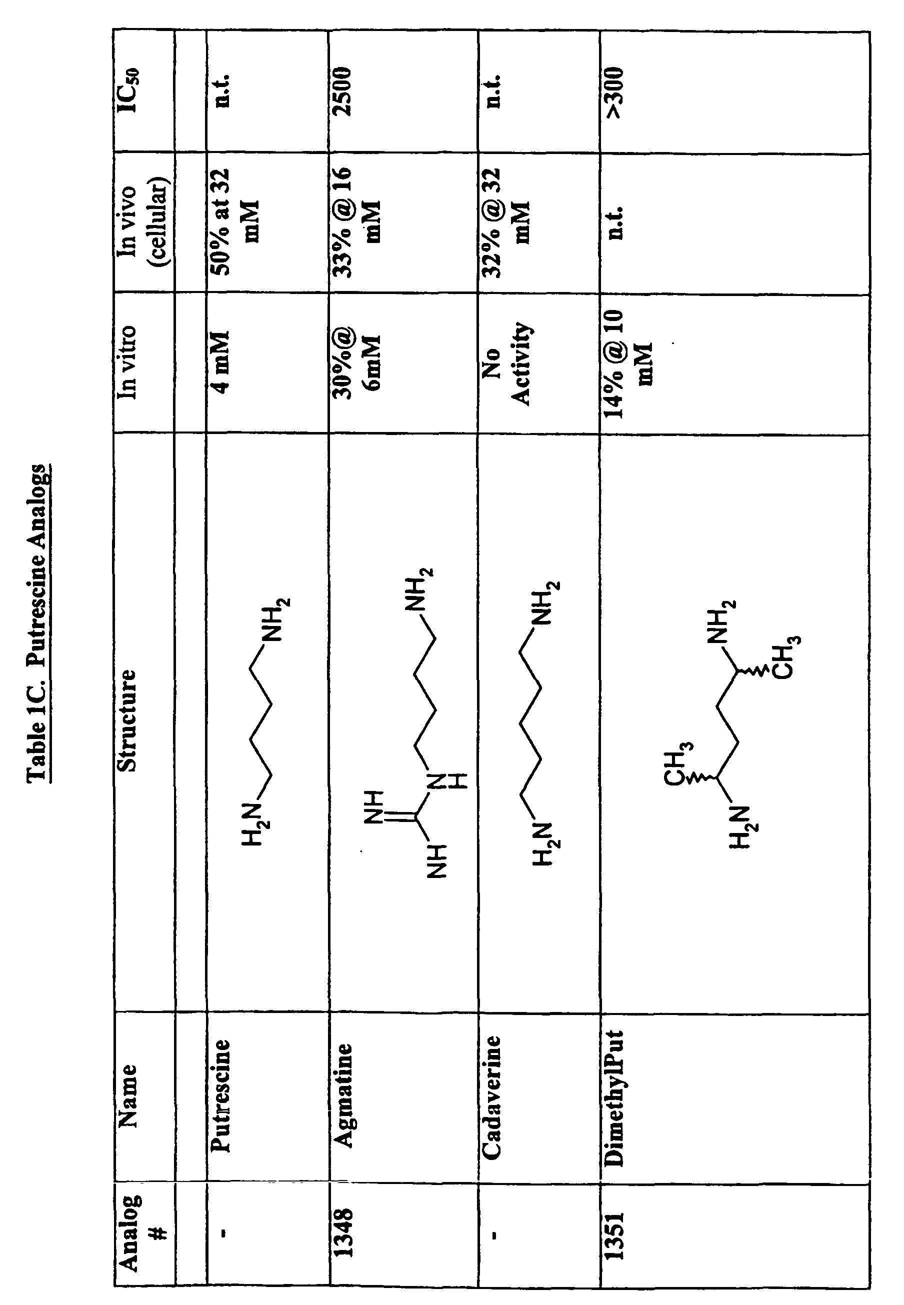

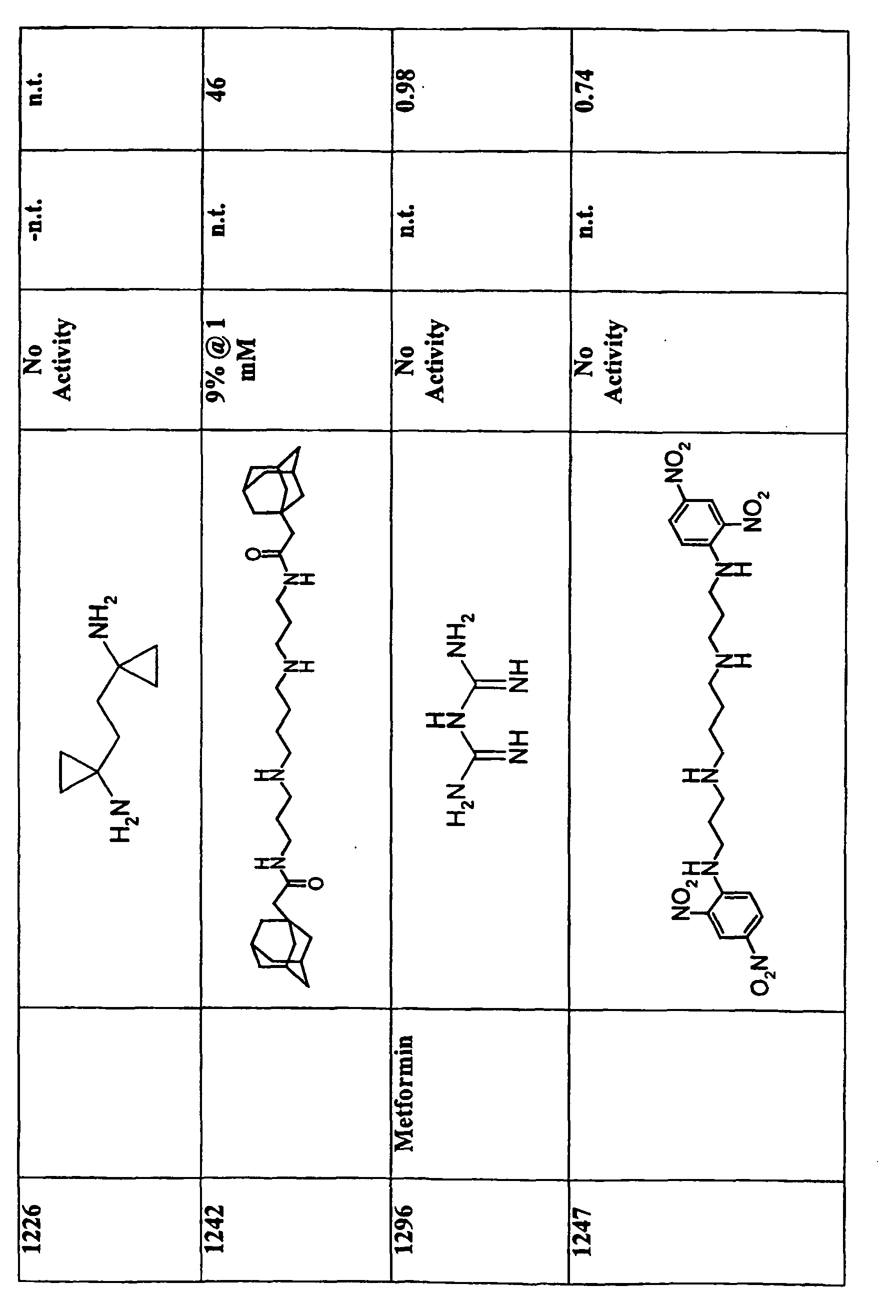

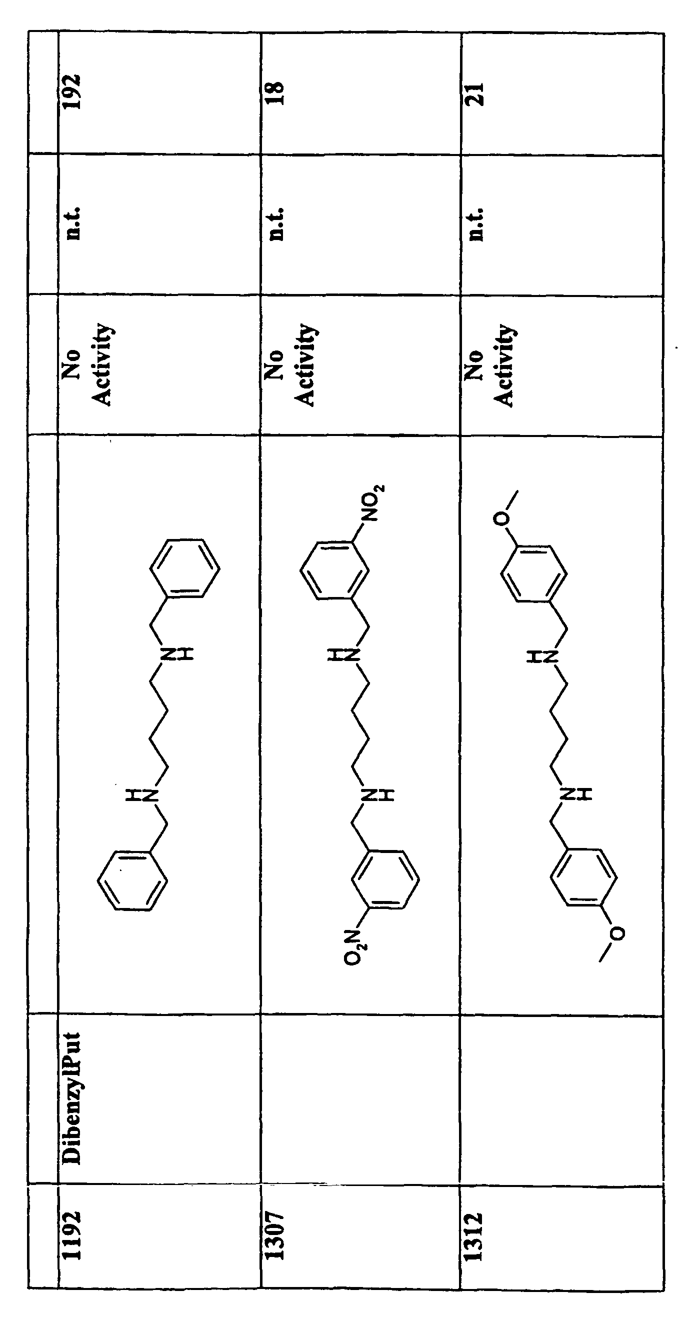

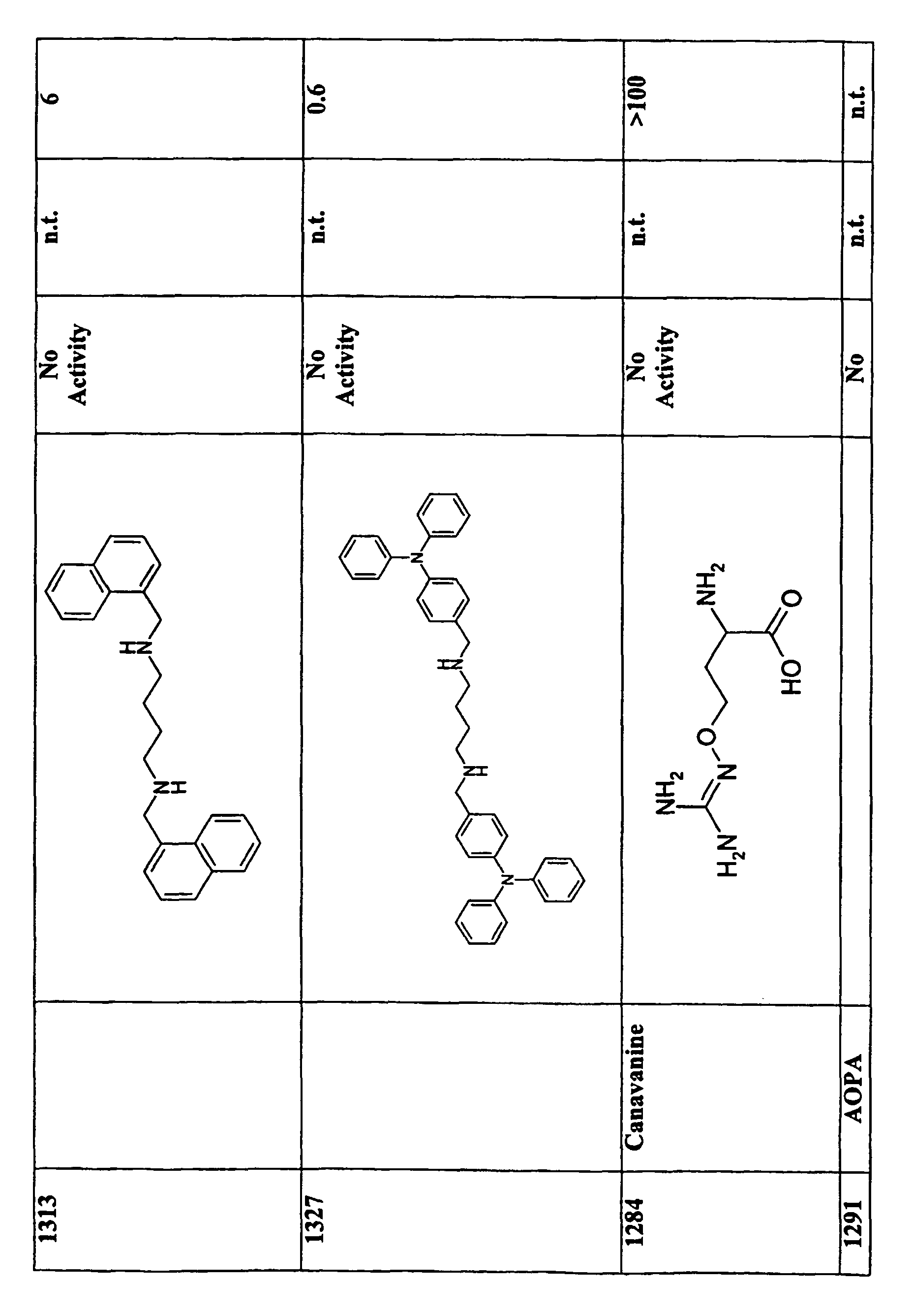

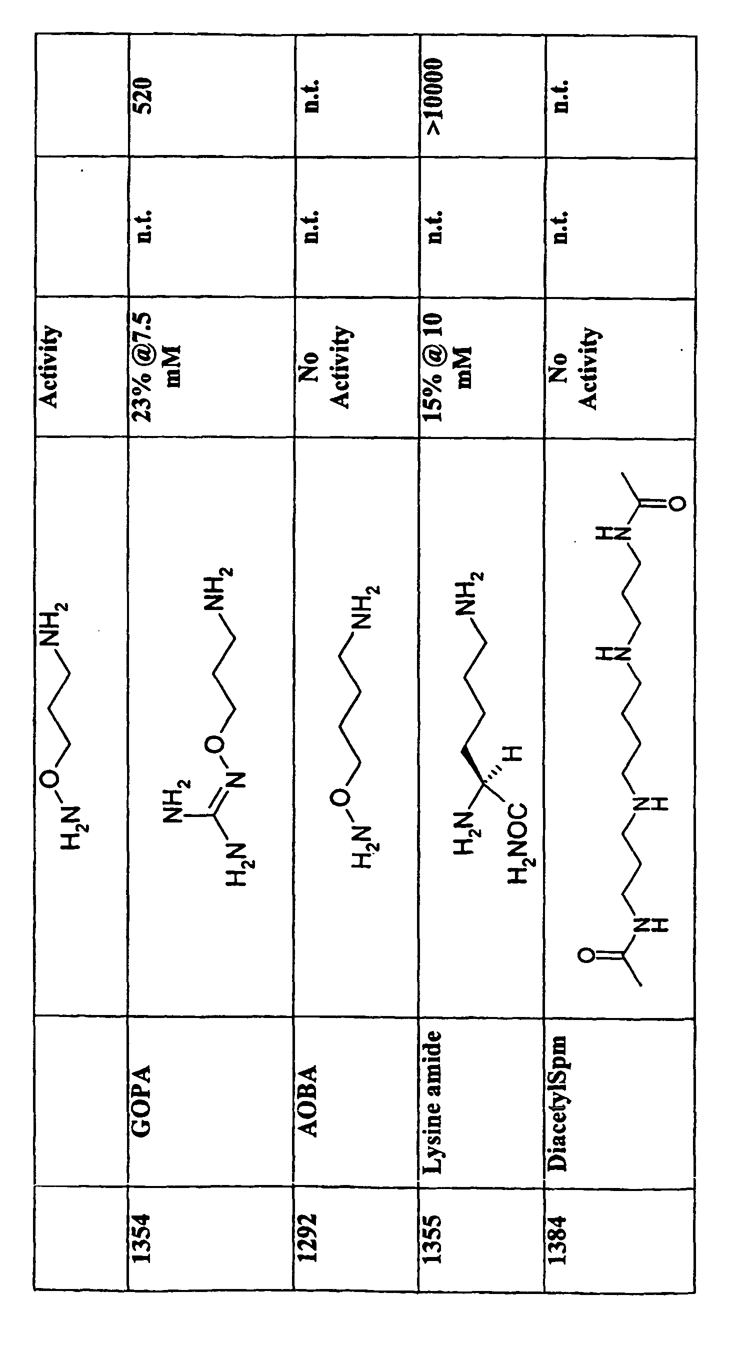

Beginning with putrescine, which has 2 positive charges, the addition of a guanidinium moiety at one end produces the agmatine compound. This change in putrescine's structure gives a molecule that is similar in activity in inducing the frameshifting of antizyme in both *in vitro* and *in vivo* (cellular) assays. Addition of an additional methylene carbon atom between the charged amino groups of putrescine results in the molecule known as cadaverine, which is only slightly less active than putrescine at inducing the antizyme +1 frameshifting event *in vivo.* Interestingly, no induction was observed in the *in vitro* assay.

Addition of two methyl groups to the terminal carbon atoms of putrescine results in analog 1351 (2, 5-diaminohexane or dimethylputrescine), which induces antizyme at least *in vitro.* The next 9 analogs in Table 1C, 1226, 1242, 1296, 1247, 1192, 1307, 1312, 1313 and 1327, contain additional substituents on the putrescine backbone. Eight of these nine analogs did not demonstrate induction activity *in vitro*, but they may nevertheless be similar to cadaverine, which also demonstrated no *in vitro* activity but significant *in vivo* activity.

The next five analogs in Table 1C (1284, 1291, 1354, 1292 and 1355) demonstrate the requirement of appropriate positive charges for antizyme induction. The natural product L-canavanine, analog 1284, is as a guanidine substituted amino acid, with a net +1 charge at pH 7.2. It showed no activity in the *in vitro* induction assay. When both the guanidinium and carboxylate groups are removed to result in analog ORI 1291 (AOPA) or its carbon-extended analog 1292 (AOBA), no induction activity is observed. Both of these analogs have a +2 net positive charge at physiological pH. Removing only the carboxylate group from L-canavanine gives rise to analog 1354 (GOPA) that is similar in *in vitro* induction activity to agmatine. This analog also contains a +2 net positive charge at physiological pH. An increase in the net positive charge of an amino acid, L-lysine, via production of its amide (net charge changed from +1 to +2) as analog 1355, results in a molecule that shows some ability to induce antizyme. This is significant since L-lysine itself does not induce antizyme activation.

Additional polyamine analogs of the invention are set forth in the following Table 2:

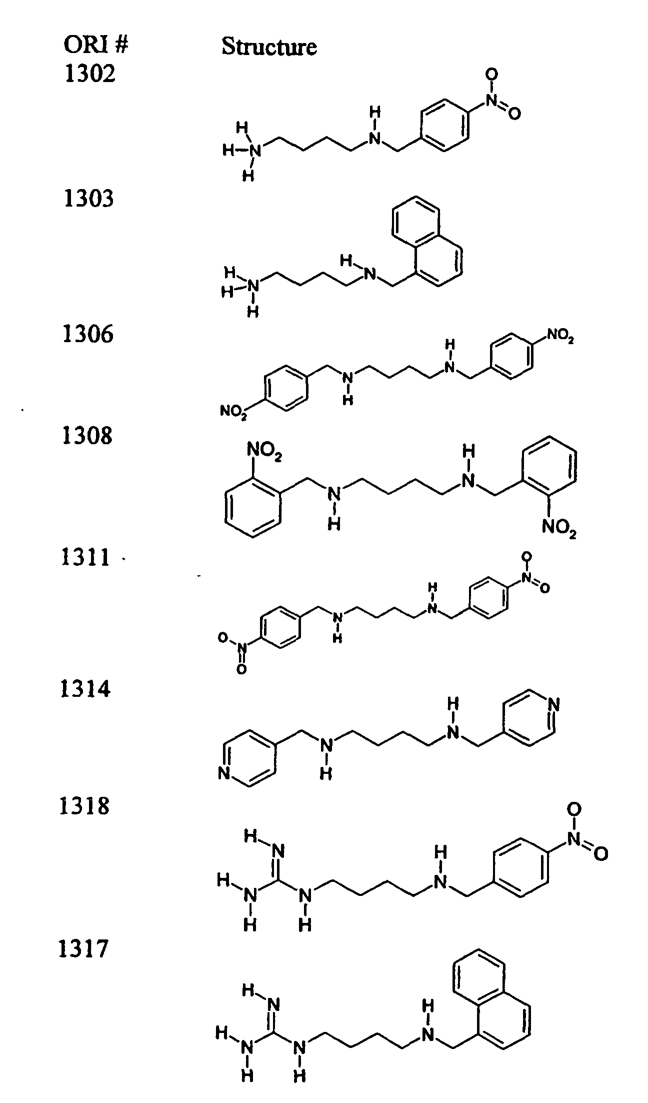

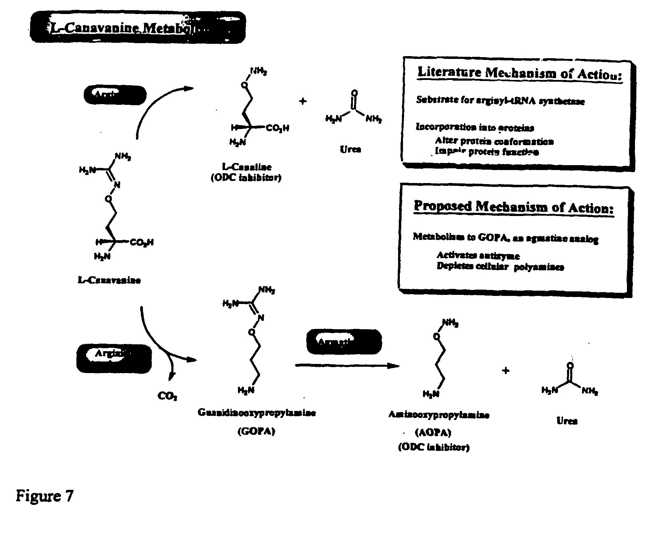

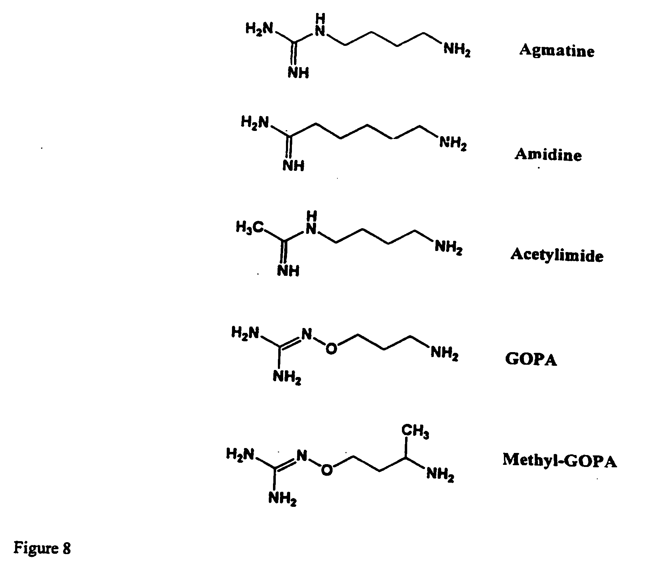

The agmatine and polyamine analogs of the invention include those that preferably cannot be metabolized to result in the generation of polyamines. Administration of agmatine, which is subject to conversion to putrescine, would be expected to defeat the goal of decreasing polyamine levels because of the likelihood of agmatinase-mediated conversion to putrescine and subsequent conversion to spermidine and spermine. Thus the invention includes the production and use of agmatine and polyamine analogs that preferably are not metabolized to putrescine or any other artificial or naturally occurring polyamine metabolite. Specific non-metabolizable analogs of agmatine and polyamine include the ethylene guanidino, which is structurally similar to GOPA and not previously described in the literature, as well as other analogs as shown in Figure 8. On the other hand, the invention encompasses the production and use of prodrug forms that are metabolized intracellularly and/or extracellularly to produce the disclosed agmatine and polyamine analogs. For example, the inventors propose that L-canavanine may be administered with the expectation that upon decarboxylation by ADC, GOPA will be generated to inhibit cell growth, as shown in Figure 7.

It is also within the scope of the invention to stabilize the disclosed agmatine and polyamine analogs by modifying them. The *in vivo* metabolic stability of a monosubstituted polyamine may be increased by modifying them to resist enzymatic degradation. For example, substitution of the terminal primary amine group with alkyl groups, or addition of alkyl groups to terminal carbon atoms of aminoalkyl groups, would achieve this goal. In addition compounds wherein secondary amino groups are alkylated are also contemplated as within the scope of the invention. N-alkylation of amide nitrogens slows down proteolytic degradation, or alternatively, the carbonyl group could be reduced to CH₂. The foregoing changes can be achieved by a number of synthetic routes. Substitution of carbon atoms α to secondary nitrogens and acylation of nitrogens may also slow degradation via the polyamine oxidase route. Such chemical modifications may also be made to minimize potential side effects of compounds when used as pharmaceuticals.

The invention further comprises agmatine and polyamine analogs substituted at one or more positions. Disubstituted agmatines are preferably substituted at the two terminal nitrogens, but may be alternatively or additionally substituted at internal nitrogen and/or internal carbon atoms. Preferred embodiments include those modified at positions in or the cyclic ends of some of the structures depicted in Figures 1-4 and 6 as well as Tables 1A-1C. In particular, modification of the six-membered ring substituents in those structurs is expected to increase binding affinity for ribosomes, tRNA or rRNA by adding more sites for interaction.

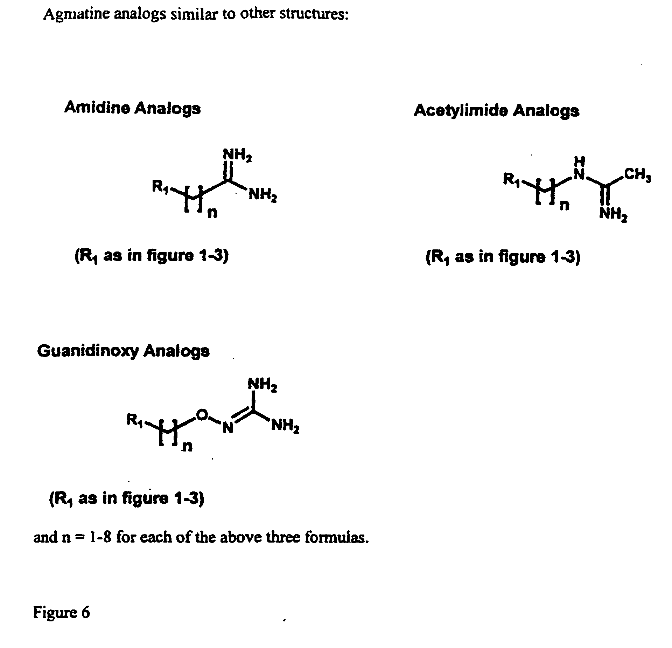

Particularly preferred embodiments are highly specific agmatine and polyamine analogs which decrease cellular polyamine levels and have pharmaceutical utility as an anti-cancer chemotherapeutic by inducing AZ. These include the compounds set forth in Figure 6 and in the above Tables.

Preferred analogs of the invention are compounds 1354, 1041 and 1283. Pharmaceutical compositions and treatment methods comprising these analogs are also preferred. The structures of other analogs that induce AZ will be compared to these compounds and each other for structural elements/motifs that are in common between them to identify structure-activity relationships involved in AZ induction based on common elements/motifs found. The structures of non-inducing analogs will also be compared to each other as well as to the preferred analogs and other AZ inducing analogs to identify common elements/motifs that are not involved in AZ induction. Thus by comparing the structures of agmatine and polyamine analogs after screens for the ability to induce AZ, structural elements/motifs both important and dispensable for AZ induction can be identified and utilized for the preparation of additional AZ inducing analogs.

For example, comparisons of the compounds discussed above have demonstrated the roles of structure, charge and separation distance between charge centers as important in the preparation of compounds for AZ induction.

### General Means of Synthesis

As one skilled in the art would appreciate, there are a multitude of means to synthesize the agmatine and polyamine analogs of the invention. One approach to synthesizing mono- and di-methyl substituted agmatine analogs is by the following approach: 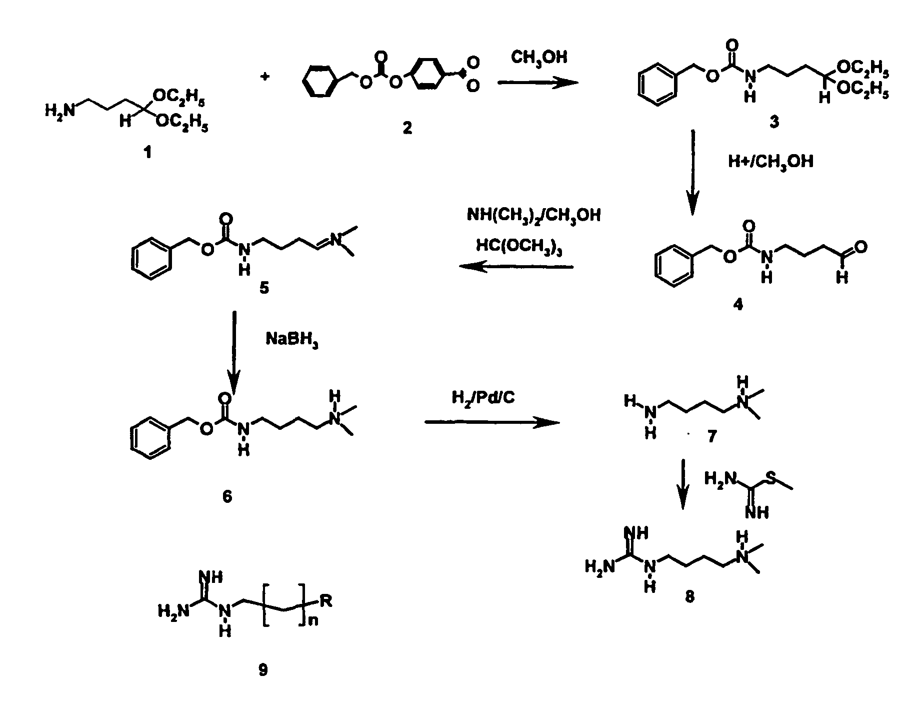 wherein in compound 9, which is a generalized form of compound 8, n = 0 to 6. This synthesis protocol is further detailed below.

Alternatively, compounds 8 and 9 may also be synthesized using an alternative synthetic approach starting from compound 10 as follows.

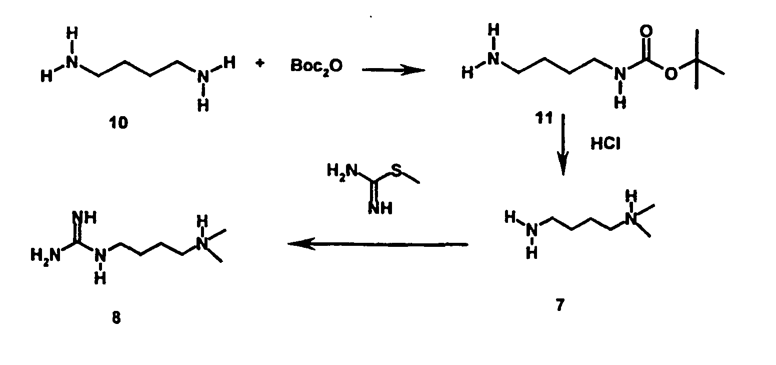

### Synthesis of L-canavanine Related Agmatine Analogs

At least two approaches to the synthesis of GOPA are readily possible. Following the chemo-enzymatic process described by Hamana, K. *et al*., (*Biochem. Biophys. Res. Comm.,* 129(1):46-51 (1985)), *E. coli* arginine decarboxylase can be used to directly produce GOPA from L-canavanine. The reaction may be followed by TLC and the resultant GOPA may be isolated by cation exchange chromatography with elution in HCl.

A second process is possible by first producing aminooxypropylamine and aminooxybutylamine via a synthetic procedure (Pankaskie, M.C. *et al., Syn. Communications,* 19(3&4):339-344 (1989)). Each of these can then be reacted with methylthioisourea to produce the desired guanidine derivatives. Since the aminooxy functionality will be much more nucleophilic than the amino function due to the alpha-effect, complete chemo-selectivity is expected for the desired product. The coupling reactions can proceed as shown below and be followed by a deprotection to set the stage for the guanidination reactions.

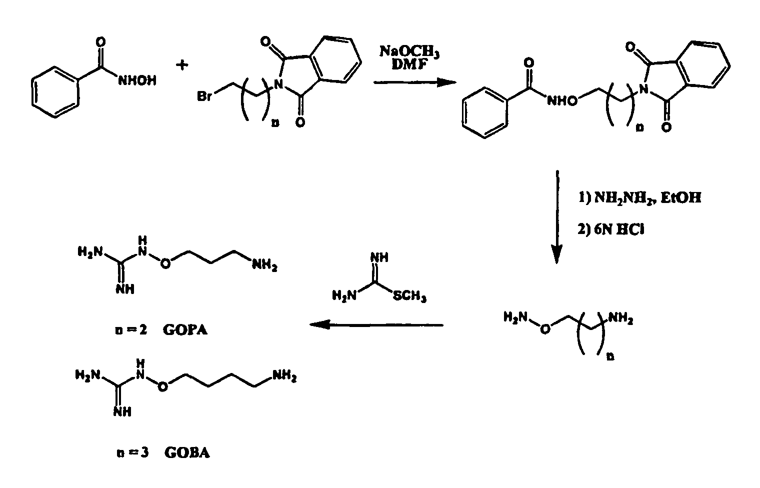

In addition, the amine oxidase resistant, α-methyl version of GOPA can be synthesized through the route shown below. Following formation of the bromophthalimide derivative the above route can be used to complete the synthesis.

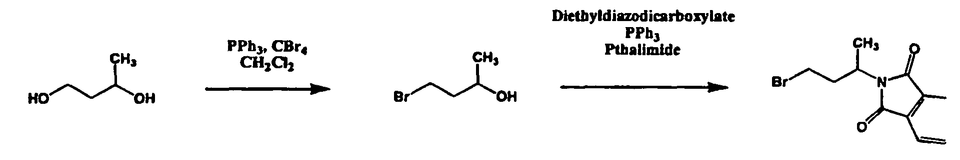

### Induction of Antizyme

As one skilled in the art would appreciate, there are a multitude of means to test the agmatine and polyamine analogs of the invention for the ability to induce a frameshift. For example, Satriano *et al*. (*J. Biol. Chem.,* 273:15313-15316 (1998)) and the references cited therein utilized an *in vitro* reticulocyte system. The agmatine and polyamine analogs of the invention were tested *in vitro* (such as by test tube) and *in vivo* (such as by cell culture) using the antizyme dual luciferase frameshifting assay as described by Grentzmann, G., *et al* in RNA 4:479-486, 1998. This assay allows one to measure the efficiency of translational frameshifting on the human antizyme sequence. Briefly, two variations of the human antizyme sequence were cloned between the renilla (5') and firefly (3') luciferase reporter genes in pRL-SV40 (Promega) plasmids. One plasmid contained the wild type antizyme sequence which requires a +1 frameshift for complete translation, the other plasmid contained modified, in-frame antizyme (control) sequences. These plasmids are utilized to transiently or stably transfect human cell lines with either the wild type or in-frame antizyme sequence-containing plasmids. Renilla and firefly luciferase activity in each assay (with cells containing one of the two plasmids) is measured to account for polymerase fall-off or translational efficiency in the presence or absence of the agmatine and polyamine analogs of the invention. That ratio is used to normalize the firefly luciferase activity seen in the wild type sequence, compared to the in-frame control sequence. The ratio can also be indicative of whether analogs of the invention completely inhibit protein translation.

*In vitro* assays of the effect of agmatine and polyamine analogs on antizyme frameshifting were performed as follows. Linearized plasmids containing the wild type and in-frame antizyme sequence were transcribed *in vitro*, followed by *in vitro* translation in reticulocyte lysate, with and without agmatine and polyamine analogs. Luminescence was measured using the Dual-Luciferase™ reporter assay (Promega).

Either the *in vivo* or *in vitro* assay may be used to screen test compounds for the ability to induce antizyme.

Alternatively, antizyme induction may be assayed by antibody based detection (Western blot) of total cellular proteins for increased levels of antizyme after treatment with an agmatine and polyamine analog (see Example III below).

### Growth Inhibition

Agmatine and polyamine analogs of the invention are selected in part based on their growth inhibitory properties, which requires inhibition of both polyamine biosynthesis and transport. The IC₅₀'s of the analogs are preferably in the low µM range or lower. When assayed over a seven day period, cells are depleted in polyamine levels due to insufficient biosynthesis and import of polyamines. While polyamine levels in the culture media may decrease initially due to residual uptake, preferred agmatine and polyamine analogs of the invention should permit the retention of residual polyamines in the media over time. Moreover, addition of polyamines, such as spermidine up to 1 µM, to the media should not be able to rescue cells from growth inhibition mediated by the instant agmatine and polyamine analogs because polyamine uptake/import will have already been inhibited by the induced antizyme. There will be residual living, but non-dividing, cells in the assay because polyamine depletion is cytostatic rather than immediately lethal. Since the growth assay alone does not necessarily verify a reduction of polyamine uptake, the growth assay and a kinetic transport assay have been used in combination to validate transport inhibition.

### Inhibition of Polyamine Transport

As one skilled in the art would appreciate, there are a multitude of means to screen for the inhibition of polyamine transport. These may be used to screen agmatine and polyamine analogs made by the various synthetic routes described above. The development of polyamine based probes and the use of transport assays of the invention has been described in U.S. applications 60/052,586, filed 7/15/97, 09/115,004, filed 7/14/98, 09/114,984, filed 7/14/98, 60/065,728, filed 11/14/97, 60/085,538, filed 5/15/98, and 09/396,523, filed 9/15/99, all of which are hereby incorporated by reference in their entireties as if fully set forth.

In the case of agmatine and polyamine analogs that lower polyamine levels only by induction of antizyme, such induction must be permitted to occur prior to any transport assay since it is antizyme, rather than the analog *per se,* that inhibits transport.

As presented in the above cited applications, the assays may be performed via the use of a fluorescently labeled probe combined with detection of cellular import by a robotic system for high throughput screening. Alternatively, chemiluminescent and colorimetric variations of the above assay as well as a conventional radiochemical assay, all described in the above applications, may be used. Agmatine and polyamine analogs identified as transport inhibitors should also show effectiveness at inducing antizyme and/or lowering intracellular polyamine levels in general. Another approach to a sensitive screening assay is the enzymatic amplification of the signal emitted by the detectable label on the polyamine used as a probe for the polyamine transporter, some of which are similar to well-known enzyme immunoassay or enzyme-linked immunosorbent assay (ELISA) and are all described in the above cited applications.

### Identification of Proteins that Bind Agmatine and Polyamine Analogs

Since agmatine has been identified as inducing the frameshift mediated expression of full-length antizyme, the agmatine and polyamine analogs of the invention which similarly induce antizyme are expected to bind the same target proteins or nucleic acids, particularly those of the ribosome, tRNA or rRNA, involved in the induction process. Thus assays for such proteins and nucleic acids are included within the scope of this invention to identify additional targets involved in the induction of antizyme or other polyamine regulating cellular factors. In addition, a tightly or irreversibly binding agmatine and polyamine analog can be used to extract and isolate any such protein or nucleic acid of interest. In such instances, the analog can be immobilized on an appropriate solid phase support which is placed in contact with material containing the cellular factors such as target proteins or nucleic acids. After removal of non-specific interactions between cellular factors and the immobilized analog, the specifically interacting factors can be isolated and analyzed. Such solid phase supports, or carriers, include glass, polystyrene, polypropylene, polyethylene, dextran, nylon, amylases, natural and modified celluloses, polyacrylamides, agaroses, and magnetite. The nature of the carrier can be either soluble to some extent or insoluble for the purposes of the present invention. The support material may have virtually any possible structural configuration so long as the immobilized analog is capable of binding to a target cellular factor. Thus, the support configuration may be spherical, as in a bead, or cylindrical, as in the inside surface of a test tube or microwell, or the external surface of a rod. Alternatively, the surface may be flat such as a sheet, test strip, microwell bottom, *etc.* Those skilled in the art will know many other suitable carriers for binding antibody or antigen, or will be able to ascertain such by use of routine experimentation.

Additionally, the analog can be conjugated to one member of a two member protein-protein interaction system (such as, but not limited to, antibody-antibody, antibody-protein A or biotin-avidin/strepavidin) before contact with cellular factors. After contact, the factor-bound conjugated analog can be isolated by the other member of the interaction system to permit isolation of the factor. Lastly, a analog can be immunoprecipitated by specific antibodies after it has bound cellular factors to facilitate isolation of the factor.

Since some proteins undergo conformational changes when a substrate or a polyamine is bound, the present screening assays are adapted so that the analog used as a probe either binds or does not bind to a protein when the latter is in a given conformational state.

### Localization of Cellular Factors Involved in Antizyme Induction

Agmatine and polyamine analogs may also be used for histochemical or cytochemical localization of cellular factors involved in the induction of antizyme. After uptake by the cell, the analogs can be used as probes (after suitable labeling by means such as, but not limited to, radioactive, fluorescent or biotinylation) in cytochemical analyses of cells or tissues to identify cells or sites within cells where antizyme induction is occurring. For example, fluorescently labeled analogs can be incorporated into traditional cytological analysis with the use of a fluorescence microscope to enhance the accuracy of current diagnostic techniques.

### Optimization of Antizyme Inducing Structural Elements/Motifs

As one skilled in the art would appreciate, there are a multitude of means to test for structural elements favorable to antizyme inducing activity. Once an activity is detected such as in analogs 1354, 1041 and 1283, substitution on the compounds are performed containing either electronic donating or electron withdrawing groups to improve activity. The influence on activity is measured and depending on the outcome, the next substituent is chosen. Similarly the effect of hydrophobic substitutions is studied. Once a number of active compounds are obtained a common pharmacophore key is determined with a program such as Chem-X (Chemical Design, San Diego). This pharmocophore key is then used to search both inhouse and commercial libraries for compounds containing the pharmacophor key to be evaluated as potential antizyme inducers.

Alternatively commercially available libraries are screened for antizyme inducing activity. Lead compounds are identified and optimized structurally and electronically to give increased antizyme inducing activity. Additional iteration of the process described above are performed to optimize a lead.

An additional means to optimize an identified lead analog such as 1354, 1041 and 1283 is by the use of parallel synthesis and combinatorial chemistry, a rapidly changing field of molecular exploration still in its infancy. For reviews, see Lam, K.S., *Anticancer Drug Des.* 12:145-167, 1997; Salemme, F.R. *et al.; Structure* 5:319-324, 1997; Gordon, E.M. *et al., J. Med. Chem.* 37:1385-1401, 1994; Gallop, M.A. *et al*., *J. Med. Chem.* 37:1233-1251, 1994). The pharmaceutical industry has realized that the original approach of the combined synthesis of hundreds to thousands of compounds in one "flask" followed by testing and deconvoluting the results is a tedious process with many pitfalls. The more traditional approach of medicinal chemistry, that is, the synthesis and testing of one compound at a time, yields more reliable and informative results about the structure around a target. The trend in combinatorial chemistry is therefore toward synthesis of multiple compounds at once, with each in a separate container. Therefore, many have adopted this one-compound/one-well parallel synthetic approach. While many lead compounds have been generated this way, the chemistries do not necessarily lead to a molecule with the necessary drug-like characteristics.

The additional steps of lead optimization followed by incorporation of drug-like characteristics (molecular weight, hydrophilicity/hydrophobicity, transport, metabolism and stability) reduce drug development costs. Therefore it is preferred to pursue novel chemistries whereby the desired characteristics are incorporated into the initial libraries. By decreasing the number of steps between lead identification and production of a new drug candidate, costs can be significantly reduced.

For example, using parallel synthesis starting with an array of substrates related to the substrates necessary for the synthesis of 1354, 1041 and 1283, a library of substituted compounds can be synthesized. Using the appropriate available equipment as many as 24 compounds can be synthesized at the same time on one instrument. Commercially available instruments support high throughput parallel synthesis. Microtiter plate formats for synthesis of 96 compounds at a time are now routine, the 96 multipin technology developed by Chiron Mimotopes PTY LTD (San Diego) being a typical example. This format is especially useful to synthesize putrescine analogs with different length carbon linkers and substitutions on the nitrogen atoms.

The synthetic approaches used to synthesize series A, B and C in Example II below can be modified to be used to synthesize libraries of compounds in parallel. Methods are known in the art to use a solution phase approach where reaction conditions are chosen such that impurities and unreacted material are separated from the desired product(s) by extraction. Alternatively a solid support approach can be used where the reaction is performed in a stepwise fashion on solid support and excess reagents are washed away and product is cleaved and recovered from the solid support.

### Applications and Uses

Due to the involvement of polyamines in numerous cellular processes, reductions in polyamine levels made possible by the analogs of the invention are useful in affecting a variety of cellular components and processes. Among these is the decrease in vascularization of solid tumors upon inhibition of polyamine synthesis. One month of treatment with DFMO resulted in a 50% reduction in neoplastic vessel count in humans with cervical interepithelial neoplasia (Mitchell, M.F. *et al., Proceedings AACR* 39:Ab. 600, (1998)). Other studies showed that DFMO inhibited the neovascularization induced by tumor cells *in vivo* (Jasnis, M.A. *et al., Cancer Lett* 79:39-43, (1994)). Thus decreases in polyamine levels made possible by the instant invention would be expected to inhibit the vascularization of solid tumors.

Another application of the analogs of the invention is with regard to restenosis. The analogs can be attached to stents in a variety of ways known in the art including covalently or as a slow release preparation to inhibit the initiation of cell proliferation after injury resulting from the procedure. In this context, the analogs of the invention may exert their beneficial effects partly or wholly by inhibiting nitric oxide synthetase (NOS), which is linked to increased levels of NO in various diseases. Various guanidino compounds, including agmatine, have been assayed for their effects on NOS (see Yokoi, I. *et al.,* In: *Guanidino Compounds in Biology and Medicine: 2,* De Deyn, P.P. et al., Eds., John Libbey & Co., London, England, 1997, pp.3-7). Diseases in which the inhibition of NOS would be expected to be of benefit include septic shock, arthritis, stroke, chronic inflammation, angiogenesis in tumors, and diabetes.

Because inhibitors of the polyamine transporter (PAT) can contribute to inhibition of cell growth, lower polyamine levels are viewed by the present inventors as being useful in the treatment of post-angioplasty injury. Endothelial denudation and vessel wall injury lead to neointimal hyperplasia and luminal stenosis. Inhibition of smooth muscle cell proliferation, for example, would be expected to inhibit neointimal formation. This initiation of cell proliferation after injury would be amenable to treatment by decreasing polyamine levels according to this invention (Takagi, M.M. *et al., Arterioscler. Thromb. Vasc. Biol.* 17*:*3611-3619, (1997); Nakaoka, T. *et al., J. Clin. Invest*. 100:2824-2832, (1997); Maillard, *L. et al., Cardiovasc. Res.* 35:536-546, (1997); Endean, E.D. et al., J. Surg. Res., 50:634-637 (1991)).

Another area expected to be affected by lower polyamine levels is rheumatoid arthritis (RA), which is commonly treated with low dose methotrexate. The reason for methotrexate's efficacy is unknown, although it is not believed to inhibit proliferation of lymphoid cells. While S-adenosylmethionine (AdoMet) metabolism has been proposed to play a direct role in the immunosuppressive activity of methotrexate, the direct effect of AdoMet metabolism on the immune response is not known, although a role for polyamines has been suggested (Furumitsu, Y. *et al., J. Rheumatology,* 20:1661-1665, (1993); Nesher, G. *et al., Arthr. Rheumat.* 33:954-957, (1990)). Moreover, cytokines are believed to play a direct role in the pathogenesis of RA, and polyamine inhibitors were observed to reverse low IL-2 levels in patients' synovial fluid (Flesher, E. *et al., J. Clin. Invest*. 83:1356-1362, (1987)). Polyamine levels are elevated in the urine, synovial fluid, synovial tissue, and peripheral blood mononuclear cells of RA patients. Culturing these cells in the presence of methotrexate inhibited the production of IgM-rheumatoid factor. Given the probable role of polyamines or elevated polyamine levels in the pathogenesis of RA, decreases in polyamine levels would be expected to have potential in lowering polyamine levels in patients with RA and thus ameliorating its effects. Additionally, the polyamine synthesis inhibitor DFMO prolongs the life of MRL-*lpr*/*lpr* mice, a model of systemic lupus erythematosus, another autoimmune disease.

A cellular component expected to be affected by lower polyamine levels is the protein eIF-5A, which appears to play a role in protein synthesis, although its exact function remains obscure (Hanauske-Abelm, H. M. *et al*., *FEBS Lett.* 266:92-98, (1995)). EIF-5A is unique in that it is modified by the unusual amino acid hypusine, which is generated post-translationally by the sequential action of deoxyhypusyl synthase (using spermidine as a substrate) and deoxyhypusyl hydroxylase. Inhibition of this modification of eIF-5A, which occurs in most, if not all, eukaryotes, coincides with proliferative arrest late in the G1 phase of the cell cycle. Thus lower polyamine levels, which would prevent post-translational modification with hypusine, would be useful in treating diseases associated with unwanted cell proliferation, such as cancer, by blocking the cell cycle.

Another means of affecting the cell cycle by lowering polyamines has been noted by the present inventors based on the observation that CHENSpm is a polyamine analogue that induces apoptosis (Ha, H.C. *Proc Nat.AcadSci., USA* 94:11557-11562 (1997)). CHENSpm also reduces spermidine and spermine levels and produces a G₂ cell cycle arrest at subtoxic concentrations, suggesting an unusual mode of action. Polyamines are known to bind to tubulin and promote its bundling, though this is just one of several possible mechanisms by which polyamines can induce apoptosis or inhibit cell growth. Also, some microtubule associated proteins (MAPs) also bind polyamines. As such, lower polyamine levels would be expected to contribute to a G₂ cell cycle arrest as well as decreases in tubulin bundling and associations with MAPs.

Another effect of lower polyamine levels is expected for parasitic organisms, such as *Trypanososma cruzi*, which are thought to obtain the polyamines necessary for their growth from hosts rather than by *de novo* synthesis. The ODC inhibitor DFMO decreases the availability of putrescine, a precursor of spermidine and spermine synthesis. DFMO can cure *T. brucei* infection in mice and is active against African sleeping sickness in humans caused by *T. brucei gambiense*. DFMO also has clinical utility in *Pneumocystis carinii* pneumonia and in infection by the coccidian protozoan parasite, *Cryptosporidium.* In the laboratory, DFMO acts against *Acanthamoeba, Leishmania, Giardia, Plasmodia* and *Eimeria* (Marton, L.J. *et al., Annu. Rev. Pharmacol. Toxicol.* 35:55-91, (1995)). Polyamines are also essential for the growth of *Hemophilus* and *Neisseria* organisms (Cohen, *S.S., A Guide to the Polyamines,* Oxford University Press, NY. pp 94-121, (1998)). Thus decreases in polyamine levels made possible by the invention would be expected to have potential in preventing, treating and/or curing infection by parasitic organisms such as *Trypanososma cruzi, T. brucei, Pneumocystis carinii, Cryptosporidium, Acanthamoeba, Leishmania, Giardia, Plasmodia, Eimeria, Hemophilus* and *Neisseria* by lowering polyamine levels necessary for their growth.

Lowering of polyamine levels may also protect plants against a wide range of fungi, *e.g., Uremyces phaseoli* Linnaeus, race O. Unifoliolate (bean rust). DFMO is an effective fungicide in the following plants: tomato plants against *Verticillium* wilt fungus; wheat against stem rust fungus and powdery mild fungus; bean plants against powdery mildew fungus; Macintosh apple leaves against the powdery mildew fungus; Ogle oats against leaf rust fungus; and corn against the corn rust fungus (U.S. Pat. 4,818,770). The analogs of the invention that lower polyamine levels would be expected to have potential in protecting plants against a wide range of fungi.

Since low polyamine levels can lead to cell death, the analogs of the invention may be used in combination with cytotoxic drugs to inhibit the growth of tumor cells. The agent 8-chloro-cAMP (8-Cl-cAMP) (Tortora *et al., Cancer Res.* 57:5107-5111 (1997)) is a cAMP analog that selectively down-regulates PLA-1, a signaling protein that is directly involved in cell proliferation and neoplastic transformation and mediates the mitogenic effects of certain oncogenes and growth factors. In nude mice bearing human GEO colon cancer xenografts, 8-Cl-cAMP inhibited tumor angiogenesis and secretion of growth factors of the EGF family and synergized with anti-EGF receptor antibodies in inhibiting tumor growth. 8-Cl-cAMP acts by different mechanisms than the agmatine and polyamine analogs of the invention, which can be used in combination with 8-Cl-cAMP, or in combination with an ODC inhibitor and/or a SAM decarboxylation inhibitor (with or without 8-Cl-cAMP). Because lower polyamine levels affects chromatin structure, other agents can be used in combination with the analogs of the invention. These include topoisomerase inhibitors, DNA alkylating agents and DNA intercalating agents such as doxorubicin, adriamycin, chlorozotocin, *etc*.

Moreover, since agmatine has a structure similar to that of metformin, a Type II diabetic drug, and has been shown to be a weak insulin secretagogue, the analogs of the instant invention would be expected to be useful in the treatment of diabetes.

### Pharmaceutical and Therapeutic Compositions and Their Administration

The compounds that may be employed in the pharmaceutical compositions of the invention include all of those agmatine and polyamine analogs described herein, as well as the pharmaceutically acceptable salts of these compounds. Pharmaceutically acceptable acid addition salts of the compounds of the invention which contain basic groups are formed where appropriate with strong or moderately strong, non-toxic, organic or inorganic acids in the presence of the basic amine by methods known to the art. Exemplary of the acid addition salts that are included in this invention are maleate, fumarate, lactate, oxalate, methanesulfonate, ethanesulfonate, benzenesulfonate, tartrate, citrate, hydrochloride, hydrobromide, sulfate, phosphate and nitrate salts.

As stated above, the compounds of the invention possess the ability to lower cellular polyamine levels and/or induce antizyme, properties that are exploited in the treatment of any of a number of diseases or conditions, preferably cancer,. A composition of this invention may be active *per se*, or may act as a "pro-drug" that is converted *in vivo* to the active form.

The compounds of the invention, as well as the pharmaceutically acceptable salts thereof, may be incorporated into convenient dosage forms, such as capsules, impregnated wafers, tablets or injectable preparations. Solid or liquid pharmaceutically acceptable carriers may be employed.

Preferably, the compounds of the invention are administered systemically, e.g., by injection. When used, injection may be by any known route, preferably intravenous, subcutaneous, intramuscular, intracranial or intraperitoneal. Injectables can be prepared in conventional forms, either as solutions or suspensions, solid forms suitable for solution or suspension in liquid prior to injection, or as emulsions.

Solid carriers include starch, lactose, calcium sulfate dihydrate, terra alba, sucrose, talc, gelatin, agar, pectin, acacia, magnesium stearate and stearic acid. Liquid carriers include syrup, peanut oil, olive oil, saline, water, dextrose, glycerol and the like. Similarly, the carrier or diluent may include any prolonged release material, such as glyceryl monostearate or glyceryl distearate, alone or with a wax. When a liquid carrier is used, the preparation may be in the form of a syrup, elixir, emulsion, soft gelatin capsule, sterile injectable liquid (e.g., a solution), such as an ampoule, or an aqueous or nonaqueous liquid suspension. A summary of such pharmaceutical compositions may be found, for example, in *Remington's Pharmaceutical Sciences,* Mack Publishing Company, Easton Pennsylvania (Gennaro 18th ed. 1990).

The pharmaceutical preparations are made following conventional techniques of pharmaceutical chemistry involving such steps as mixing, granulating and compressing, when necessary for tablet forms, or mixing, filling and dissolving the ingredients, as appropriate, to give the desired products for oral or parenteral, including , topical, transdermal, intravaginal, intranasal, intrabronchial, intracranial, intraocular, intraaural and rectal administration. The pharmaceutical compositions may also contain minor amounts of nontoxic auxiliary substances such as wetting or emulsifying agents, pH buffering agents and so forth. Pharmaceutical compositions formulated for timed release may also be prepared.

Although the preferred routes of administration are systemic, the pharmaceutical composition may be administered topically or transdermally, e.g., as an ointment, cream or gel; orally; rectally; *e.g.*, as a suppository, parenterally, by injection or continuously by infusion; intravaginally; intranasally; intrabronchially; intracranially intra-aurally; or intraocularly.

For topical application, the compound may be incorporated into topically applied vehicles such as a salve or ointment. The carrier for the active ingredient may be either in sprayable or nonsprayable form. Non-sprayable forms can be semi-solid or solid forms comprising a carrier indigenous to topical application and having a dynamic viscosity preferably greater than that of water. Suitable formulations include, but are not limited to, solution, suspensions, emulsions, creams, ointments, powders, linirnents, salves, and the like. If desired, these may be sterilized or mixed with auxiliary agents, e.g., preservatives, stabilizers, wetting agents, buffers, or salts for influencing osmotic pressure and the like. Preferred vehicles for non-sprayable topical preparations include ointment bases, e.g., polyethylene glycol-1000 (PEG-1000); conventional creams such as HEB cream; gels; as well as petroleum jelly and the like.

Also suitable for topic application are sprayable aerosol preparations wherein the compound, preferably in combination with a solid or liquid inert carrier material, is packaged in a squeeze bottle or in admixture with a pressurized volatile, normally gaseous propellant. The aerosol preparations can contain solvents, buffers, surfactants, perfumes, and/or antioxidants in addition to the compounds of the invention.

For the preferred topical applications, especially for humans, it is preferred to administer an effective amount of the compound to a target area, e.g., skin surface, mucous membrane, eyes, *etc.* This amount will generally range from about 0.001 mg to about 1 g per application, depending upon the area to be treated, the severity of the symptoms, and the nature of the topical vehicle employed.

The compositions of the invention be given in combination with one or more additional compounds that are used to treat the disease or condition. For treating cancer, the agmatine and polyamine analogs are given in combination with anti-tumor agents, such as mitotic inhibitors, *e.g.*, vinblastine; alkylating agents, *e*.*g*., cyclophosphamide; folate inhibitors, *e*.*g*., methotrexate, pritrexim or trimetrexate; antimetabolites, *e*.*g*., 5-fluorouracil and cytosine arabinoside; intercalating antibiotics, *e*.*g*., adriamycin and bleomycin; enzymes or enzyme inhibitors, *e.g.*, asparaginase; topoisomerase inhibitors, *e.g.*, etoposide; or biological response modifiers, *e.g.*, interferon. In fact, pharmaceutical compositions comprising any known cancer therapeutic in combination with the agmatine and polyamine analogs disclosed herein are within the scope of this invention.

The composition of the invention may also comprise one or more other medicaments such as anti-infectives including antibacterial, anti-fungal, antiparasitic, anti-viral, and anti-coccidial agents.

Typical single dosages of the compounds of this invention are between about 1ng and about 10g/kg body weight. The dose is preferably between about 0.01mg and about 1g/kg body wt. and, most preferably, between about 0.1mg and about 100mg/kg body wt. For topical administration, dosages in the range of about 0.01-20% concentration of the compound, preferably 1-5%, are suggested. A total daily dosage in the range of about 1-500 mg is preferred for oral administration. The foregoing ranges are, however, suggestive, as the number of variables in regard to an individual treatment regime is large, and considerable excursions from these recommended values are expected.

Effective amounts or doses of the compound for treating a disease or condition can be determined using recognized *in vitro* systems or *in vivo* animal models for the particular disease or condition. In the case of cancer, many art-recognized models are known and are representative of a broad spectrum of human tumors. The compounds may be tested for inhibition of tumor cell growth in culture using standard assays with any of a multitude of tumor cell lines of human or nonhuman animal origin. Many of these approaches, including animal models, are described in detail in Geran, R.I. *et al*., "Protocols for Screening Chemical Agents and Natural Products Against Animal Tumors and Other Biological Systems (Third Edition)", *Canc. Chemother. Reports,* Part 3, 3:1-112.

Having now generally described the invention, the same will be more readily understood through reference to the following examples which are provided by way of illustration, and are not intended to be limiting of the present invention, unless specified.

### EXAMPLES

### EXAMPLE I

### Synthesis of Agmatine Analogs

One approach to synthesizing mono- and di-methyl substituted agmatine analogs has been presented above and is reproduced below: 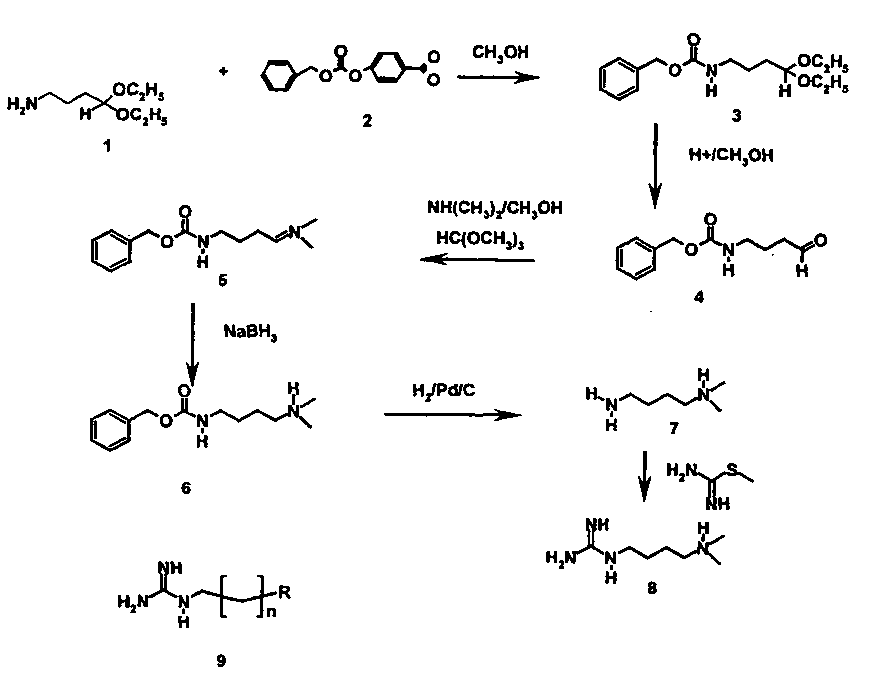 wherein in compound 9, which is a generalized form of compound 8, n = 0 to 6.

Starting with different aminoalkyldiethylacetals and different amines, a number of derivatives can be synthesized. A number of examples are shown in Figures 1-4 and 6. Compound 10 (below) is synthesized using the above approach, starting with 2-aminoethyldiethylacetal. Compound 11 can be synthesized using the same approach starting with 3-aminopropyldiethylacetal and using pyrrolidine instead of dimethylamine in the synthesis of intermediate compound 5.

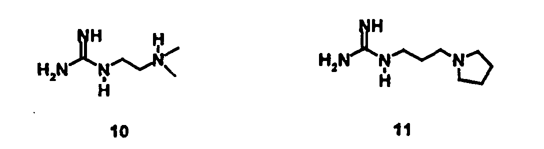

### EXAMPLE II

### Synthesis of Diaminoalkane Derivatives

Series A, B, and C below can be synthesized using the following route. Each series was produced in a parallel synthetic method and obtained in a pure form by crystallization or column chromatography over silica gel or Dowex 50 cation exchange resin.

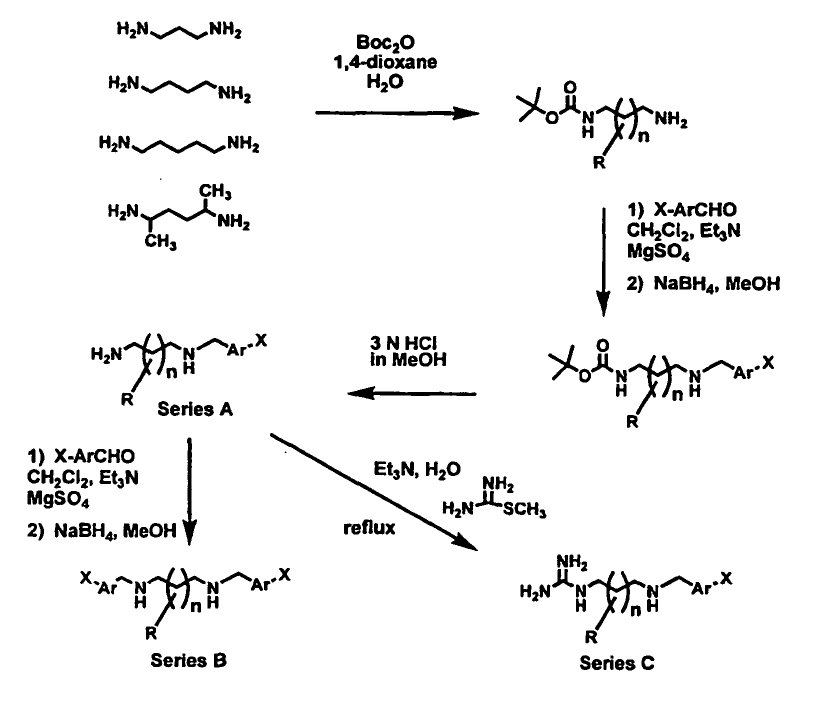

Series B with the same arylalkyl grouping on both sides can also be synthesized using the following scheme:

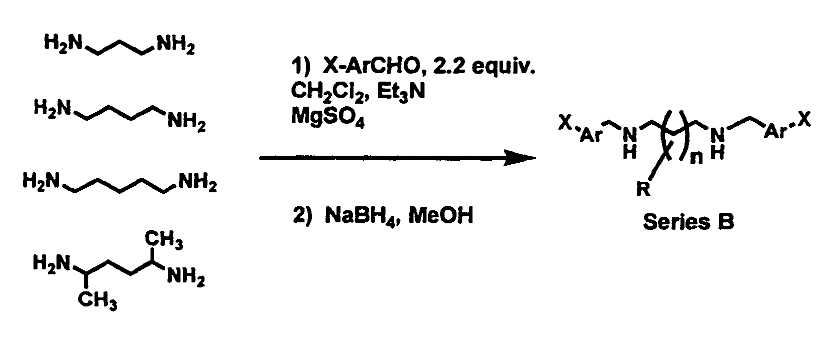

The initial series of compounds used the following aromatic and aliphatic aldehydes. This series can be greatly extended through use of the many hundreds of aldehydes available commercially. Series A is identical to compounds 8 and 9 in Example I above.

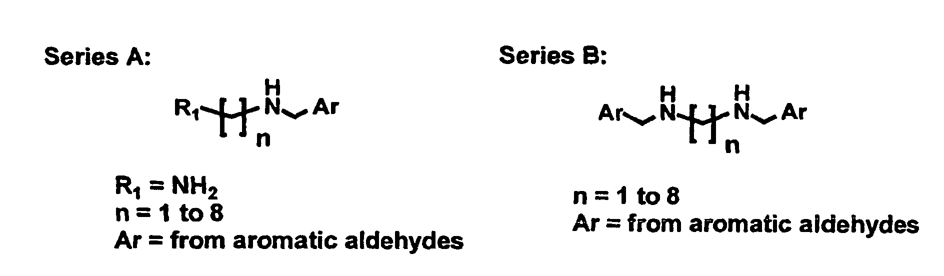

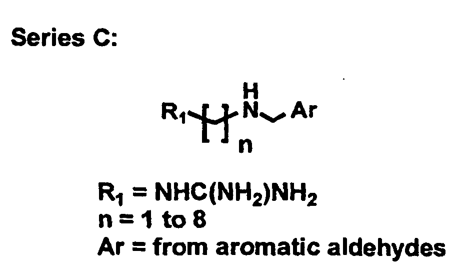

An additional group of analogs, Series D, containing two different substituents on the ammo groups of the diaminoalkane, can be synthesized directly from Series A. By choosing a different aldehyde, a very large series of unsymmetric disubstituted analogs is produced. For example, a series D can be synthesized when an X-Ar₂CHO is substituted for the generic X-ArCHO in the series B synthetic scheme shown above. Thus a large number of unsymmetric disubstituted diamine analogs can be produced by using different diamines and different aldehydes. This is schematically shown by the following, where Ar₁ represents the Ar moiety in Series A shown above. In the interest of clarity, Ar₁ and Ar₂ represent different aromatic groups rather than monomeric and dimeric forms of the same group.

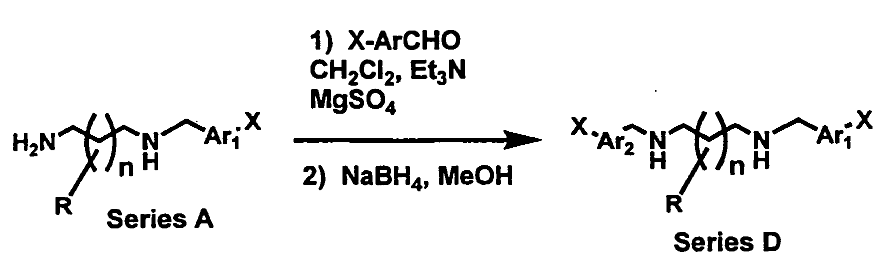

More analogs are produced by coupling of two equivalents of carboxylic acid with the diaminoalkane to produce a diamide intermediate. This intermediate is then reduced to the methylene oxidation state by using BH3.THF. Several representative carboxylic acids are shown.

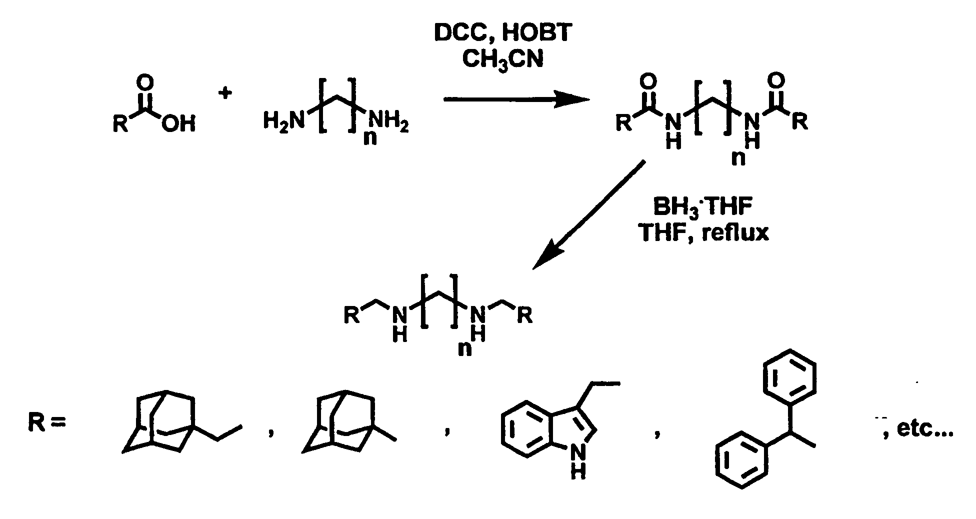

An series of reactive analogs which incorporate aziridine functionalities can be produced through the following route. Synthesis of the diketone starting material followed the method used by Sudweeks et al (J. Org. Chem. 1975, 40(8), 1131-1136.). Formation of the dioxime is followed by treatment with lithium alumnium hydride to give the diaziridine derivative shown.

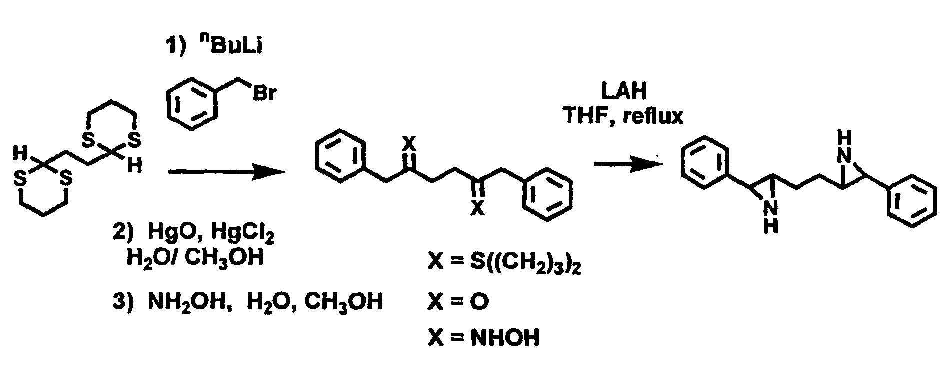

### EXAMPLE III

### Antizyme Frameshifting Assays

Agmatine and polyamine analogs of the invention were tested *in vitro* (such as by test tube) and *in vivo* (such as with cell lines) using the antizyme dual luciferase frameshifting assay as described by Grentzmann, G., *et al* in RNA 4:479-486, 1998. This assay allows one to measure the efficiency of translational frameshifting on the human antizyme sequence. Briefly, two variations of the human antizyme sequence were cloned between the renilla (5') and firefly (3') luciferase reporter genes in pRL-SV40 (Promega) plasmids. One plasmid contained the wild type antizyme sequence which requires a +1 frameshift for complete translation, the other plasmid contained modified, in-frame antizyme (control) sequences. These plasmids are utilized to transiently or stably transfect human cell lines with either the wild type or in-frame antizyme sequence-containing plasmids. Renilla and firefly luciferase activity in each assay (with cells containing one of the two plasmids) is measured to account for polymerase fall-off or translational efficiency in the presence or absence of the analogs of the invention. That ratio is used to normalize the firefly luciferase activity seen in the wild type sequence, compared to the in-frame control sequence.

*In vitro* assays of the effect of agmatine analogs on antizyme frameshifting were performed as follows. Linearized plasmids containing the wild type and in-frame antizyme sequence were transcribed *in vitro*, followed by *in vitro* translation in reticulocyte lysate, with and without agmatine analogs . Luminescence was measured using the Dual-Luciferase™ reporter assay (Promega).

### EXAMPLE IV

### Cell Growth and its Inhibition by Agmatine and Polyamine Analogs

Cells were plated in 96-well plates such that they would be in log growth for the duration of the assay. The day after plating, drugs were added to the cells and they were grown for seven days. Cell growth was measured by MTS/PMS dye assay (Promega). The assay was done in the presence of 1 mM aminoguanidine to inhibit amine oxidases from the serum in the culture media and 1 µM spermidine to insure that any growth inhibition was not the result of depletion of external polyamines from the media. The assay was also performed over seven days because this allows for the greatest dynamic range in cell growth due to the mechanism of concomitant inhibition of polyamine biosynthesis and transport. Cells need to divide several times before the intracellular level of polyamines begin to decrease to growth inhibitory levels. Therefore, growth does not significantly cease until the third to fourth day.

### EXAMPLE V

### Screening of Agmatine and Polyamine Analogs in Transport Assays

Cells in log growth are pre-incubated with an agmatine analog followed by addition of ³H-spermidine. The cells were incubated 10 min. at 37°C, which had previously been shown to be within the linear range for polyamine uptake. The cells were then washed three times with cold PBS, lysed with 0.1% SDS, and the amount of polyamine incorporation into the cells was determined by scintillation counting of the cell lysates. To determine a Kᵢ, four substrate concentrations and five inhibitor concentrations and a control were tested. The Kᵢ was determined by a non-linear fit to the Michaelis-Menten equation.

### EXAMPLE VI

### Screening of Agmatine and Polyamine Analogs for both Transport and Growth Inhibition

For example, compounds ORI 1041 and ORI 1093 were evaluated as polyamine transport inhibitors and for growth inhibition in a cell based (MDA-MB-231 breast cancer cell line) assay with and without DFMO. The results are shown in the following Table 3.

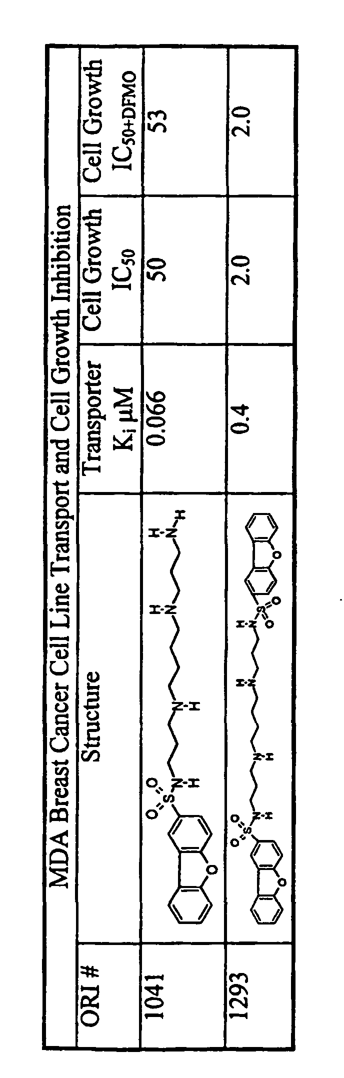

Typically compounds similar to ORI 1041 are not cytotoxic in the cell growth assay on their own until IC₅₀>300 µM. However, in the presence of DFMO the compound often shows a large decrease in the IC₅₀, measured as the R value (R= IC₅₀/ IC_{50+DFMO}). R values as high as 300 are observed. In a small subset of compounds such as ORI 1041 the R values is close to 1, meaning that the compounds are cytotoxic on their own. As observed for ORI 1041 and ORI 1093 the R values are 1 or close to 1. It is suspected that ORI 1041, a fairly good transport inhibitor, is also an antizyme inducer. ORI 1293, a disubstituted putrescine derivative, is a fairly poor transport inhibitor, but an effective cytotoxic derivative with a IC₅₀ = 2 µM. It is proposed that the toxicities shown in cell culture by these two putrescine derivatives are due to antizyme induction. Similar cell growth rates were obtained with these two compounds in PC-3 prostate cancer cell lines.

## Claims

1. Use of a polyamine analog of spermine in the preparation of a medicament for inducing expression of full-length antizyme
wherein said polyamine analog induces expression of full-length antizyme and comprises
four amine groups capable of forming four positive charges at physiological pH, wherein the first and second amine groups, and the third and fourth amine groups, are separated by the distance of four C-C and/or C-N bonds and said second and third amine groups are separated by the distance of five C-C and/or C-N bonds or more.

2. The use of claim 1 wherein said second and third amine groups are separated by -CH₂-Group-CH₂-
wherein said Group is a C₂₋₁₀, straight or branched, alkyl, alkenyl, alkynyl, alkoxy, or aliphatic; C₃₋₁₀ alicyclic; single or mults-ring aromatic or aryl; aryl-substituted alkyl, alkenyl or alkynyl; single or multi-ring aryl substituted aliphatic; aliphatic-substituted single or multi-ring aromatic; alkyl-, alkenyl-, or alkynyl-substituted aryl; a single or multi-ring heterocyclic; a single or multi-ring heterocyclic-substituted aliphatic; an aliphatic-substituted aromatic; heterocyclic-substituted alkyl, alkenyl or alkynyl; or alkyl-, alkenyl-, or alkynyl-substituted heterocyclic.

3. The use of claim 2 wherein said Group is a single or multi-ring aromatic or aryl; aliphatic-substituted single or multi-ring aromatic; alkyl-, alkenyl-, or alkynyl-substituted aryl; a single or multi-ring heterocyclic; an aliphatic-substituted aromatic; or alkyl-, alkenyl-, or alkynyl-substituted heterocyclic.

4. The use of claim 3 wherein said Group is any one of the following 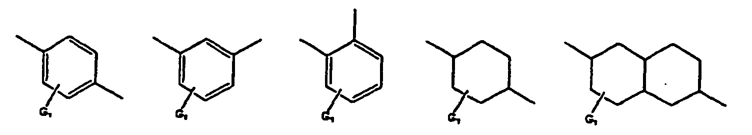 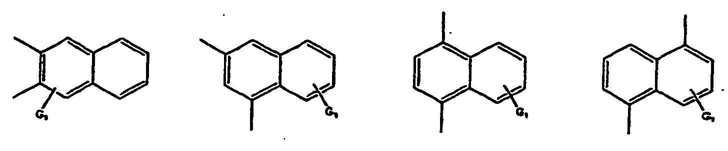 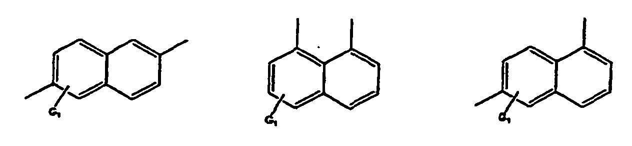 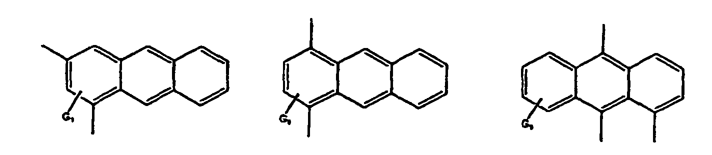 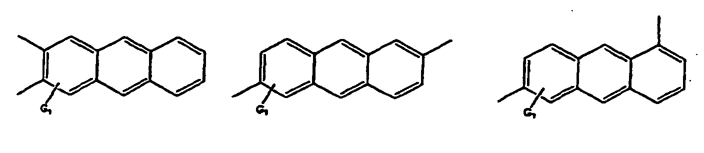 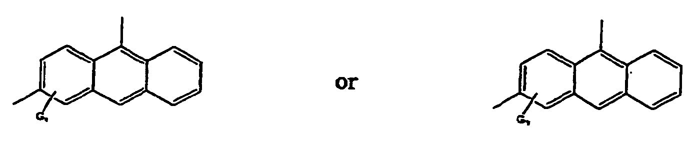 wherein G₁ is -H; a C₂₋₁₀, straight or branched, alkyl, alkenyl, alkynyl, alkoxy, or aliphatic; C₃₋₁₀ alicyclic; single or multi-ring aromatic or aryl; aryl-substituted alkyl, alkenyl or alkynyl; single or multi-ring aryl substituted aliphatic; aliphatic-substituted single or multi-ring aromatic; alkyl-, alkenyl-, or alkynyl-substituted aryl; a single or multi-ring heterocyclic; a single or multi-ring heterocyclic-substituted aliphatic; an aliphatic-substituted aromatic; heterocyclic-substituted alkyl, alkenyl or alkynyl; alkyl-, alkenyl-, or alkynyl-substituted heterocyclic; or combinations thereof.

5. The use of claim 4 wherein G₁ is -H, -CH₃, -CH₂CH₃, -CH₂Ph, -NH₂, -NHCOCH₃, -N₃, -CN, F, -CL, -BR, -I, -CF₃, -OCH₃, -OCH₂Ph, -COCH₃, -COOH, -COOCH₃, -COOCH₂CH₃, -COOCH₂Ph, -OCH₂CH₂O-, -C(NH₂)=NH, or combinations thereof.

6. The use of any one of claims 1-5 futher comprising a substituent on one or more of said amine groups or on one or more adjacent carbon atoms.

7. The use of claim 6 wherein said one or more adjacent carbon atoms are one or both of the terminal carbon atoms in spermine.

8. The use of claim 6 or 7 wherein said substituent is a C₁₋₁₀, straight or branched, alkyl, alkenyl, alkynyl, alkoxy, or aliphatic; C₃₋₁₀ alicyclic; single or multi-ring aromatic or aryl; aryl-substituted alkyl, alkenyl or alkynyl; single or multi-ring aryl substituted aliphatic; aliphatic-substituted single or multi-ring aromatic; alkyl-, alkenyl-, or alkynyl-substituted aryl; a single or multi-ring heterocyclic; a single or multi-ring heterocyclic-substituted aliphatic; an aliphatic-substituted aromatic; heterocyclic-substituted alkyl, alkenyl or alkynyl; or alkyl-, alkenyl-, or alkynyl-substituted heterocyclic.

9. The use of claim 8 wherein said substituent is methyl, ethyl, phenyl, or CH₂Ph.

10. The use of claim 9 wherein said analog is 1283.

11. Use of a polyamine analog of spermidine in the preparation of a medicament for inducing expression of full-length antizyme
wherein said polyamine analog induces expression of full-length antizyme and comprises
three amine groups capable of forming three positive charges at physiological pH, wherein the first and second amine groups are separated by the distance of five C-C and/or C-N bonds and said second and third amine groups are separated by the distance of four C-C and/or C-N bonds.

12. The use of claim 11 futher comprising a substituent on one or more of said amine groups or on one or more adjacent carbon atoms.

13. The use of claim 12 wherein said one or more adjacent carbon atoms are one or both of the terminal carbon atoms in spermidine.

14. The use of claim 12 wherein said substituent is a carboxylic amide or sulfonamide amide on the N¹-position of spermidine.

15. The use of claim 14 wherein said carboxylic amide is an alkyl or aryl carboxylic substituent comprising a C₂₋₁₀, straight or branched, alkyl, alkenyl, alkynyl, alkoxy, or aliphatic; C₃₋₁₀ alicyclic; single or multi-ring aromatic or aryl; aryl-substituted alkyl, alkenyl or alkynyl; single or multi-ring aryl substituted aliphatic; aliphatic-substituted single or multi-ring aromatic; alkyl- alkenyl-, or alkynyl-substituted aryl; a single or multi-ring heterocyclic; a single or multi-ring heterocyclic-substituted aliphatic; an aliphatic-substituted aromatic; heterocyclic-substituted alkyl, alkenyl or alkynyl; or alkyl-, alkenyl-, or alkynyl-substituted heterocyclic.

16. The use of claim 14 wherein said sulfonamide amide is an alkyl or aryl sulfonamide substituent comprising a C₂₋₁₀, straight or branched, alkyl, alkenyl, alkynyl, alkoxy, or aliphatic; C₃₋₁₀ alicyclic; single or multi-ring aromatic or aryl; aryl-substituted alkyl, alkenyl or alkynyl; single or multi-ring aryl substituted aliphatic; aliphatic-substituted single or multi-ring aromatic; alkyl-, alkenyl-, or alkynyl-substituted aryl; a single or multi-ring heterocyclic; a single or multi-ring heterocyclic-substituted aliphatic; an aliphatic-substituted aromatic; heterocyclic-substituted alkyl, alkenyl or alkynyl; or alkyl-, alkenyl-, or alkynyl-substituted heterocyclic.

17. The use of claim 15 or 16 wherein said alkyl containing substituent further contains a halide or said alkoxy moiety is methoxy, ethoxy, and substituted or unsubstituted benzyloxy.

18. The use of claim 15 wherein said analog is 1041.

19. Use of a polyamine analog of putrescine in the preparation of a medicament for inducing expression of full-length antizyme
wherein said polyamine analog induces expression of full-length antizyme, is not agmatine, and comprises
two amine groups capable of forming two positive charges at physiological pH, separated by the distance of five C-C, C-O, IV-O, and/or C-N bonds or more.

20. The use of claim 19 further comprising one or more substituents on one or more of said amine groups or on one or more adjacent carbon atoms.

21. The use of claim 20 wherein said one or more substituents is a C₁₋₁₀, straight or branched, alkyl, alkenyl, alkynyl, alkoxy, or aliphatic; C₃₋₁₀ alicyclic; single or multi-ring aromatic or aryl; aryl-substituted alkyl, alkenyl or alkynyl; single or multi-ring aryl substituted aliphatic; aliphatic-substituted single or multi-ring aromatic; alkyl-, alkenyl-, or alkynyl-substituted aryl; a single or multi-ring heterocyclic; a single or multi-ring heterocyclic-substituted aliphatic; an aliphatic-substituted aromatic; heterocyclic-substituted alkyl, alkenyl or alkynyl; or alkyl-, alkenyl-, or alkynyl-substituted heterocyclic.

22. The use of claim 21 wherein said one or more substituents is -H, -CH₃, -CH₂CH₃, and -CH₂CH₂-.

23. The use of claim 22 wherein said analog is selected from 1,4-diaminobutane with monomethyl or dimethyl substitutents in the 1, 2, 3 or 4 position; 1,4-, 1,3-, or 1,2- dimethyl-diaminobutane; bis-1,4-cyclopropyl-1,4-diaminobutane; and 1,1,4,4-tetramethyldiaminobutane.

24. The use of claim 21 wherein said analog is 1354.

25. The use of any one of the preceding claims wherein said medicament further comprises a pharmaceutically acceptable excipient

26. An in vitro method of inducing full-length antizyme expression comprising contacting an antizyme expression system with an analog of any one of the preceding claims.

27. An in vitro method of inhibiting cell growth comprising contacting said cell with an analog of any one of the preceding claims.

## Patentansprüche

1. Verwendung eines Polyamin-Analogons von Spermin bei der Herstellung eines Arzneimittels zur Induktion der Expression eines Antizyms voller Länge,
worin genanntes Polyamin-Analogon die Expression des Antizyms voller Länge induziert und Folgendes umfasst:
vier Amingruppen, die zur Bildung von vier positiven Ladungen bei physiologischem pH fähig sind, worin die ersten und zweiten Amingruppen und die dritten und vierten Amingruppen durch den Abstand von vier C-C- und/oder C-N-Bindungen getrennt sind und die genannten zweiten und dritten Amingruppen durch den Abstand von fünf C-C- und/oder C-N-Bindungen oder mehr getrennt sind.

2. Verwendung nach Anspruch 1, worin die genannten zweiten und dritten Amingruppen durch -CH₂-Gruppe-CH₂ getrennt sind,
worin genannte Gruppe Folgendes darstellt: ein(en) C₂₋₁₀, gerad- oder vezweigtkettig, Alkyl, Alkenyl, Alkynyl, Alkoxy oder Aliphaten; C₃₋₁₀-Alicyclus; Einzel- oder Mehrringaromaten oder -aryl; Aryl-substituiertes Alkyl, Alkenyl oder Alkynyl; Einzel-oder Mehrringaryl-substituierten Aliphaten; Aliphat-substituierten Einzel- oder Mehrringaromaten; Akyl-, Alkenyl- oder Alkynyl-substituiertes Aryl; einen Einzel- oder Mehrringheterocyclus; einen Einzel- oder Mehrringhcterocyclus-substituierten Aliphaten; einen Aliphat-substituierten Aromaten; Heterocyclus-substituiertes Alkyl, Alkenyl oder Alkynyl; oder Alkyl-, Akenyl- oder Alkynyl-substituierten Heterocyclus.

3. Verwendung nach Anspruch 2, worin genannte Gruppe Folgendes darstellt: ein(en) Einzel- oder Mehrringaromaten oder -aryl, Aliphat-substituierten Einzel- oder Mehrringaromaten; Alkyl-, Alkenyl- oder Alkynyl-substituiertes Aryl; einen Einzel- oder Mehrringheterocyclus; einen Aliphat-substituierten Aromaten; oder Alkyl-, Alkenyl-, oder Alkynyl-substituierten Heterocyclus.

4. Verwendung nach Anspruch 3, worin genannte Gruppe jedwede eine der folgenden 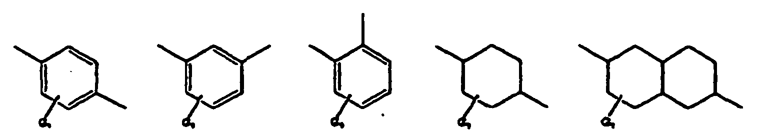 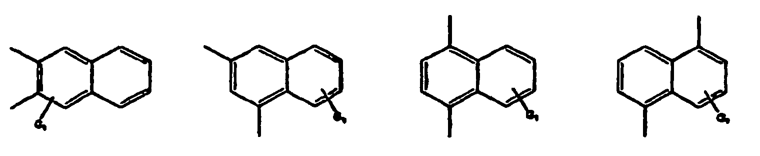 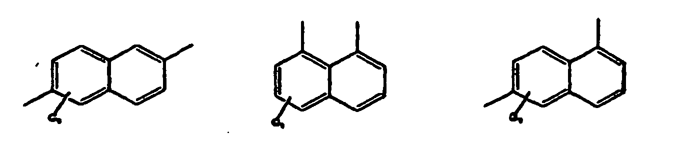 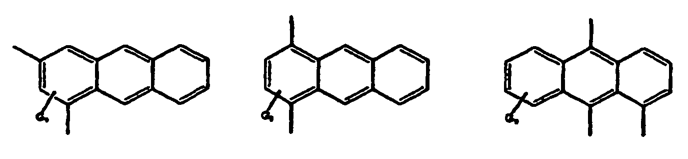 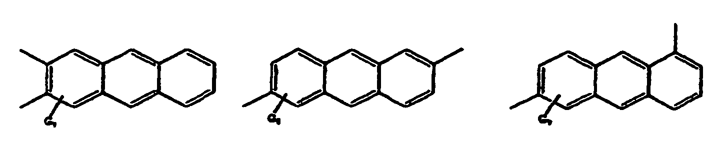 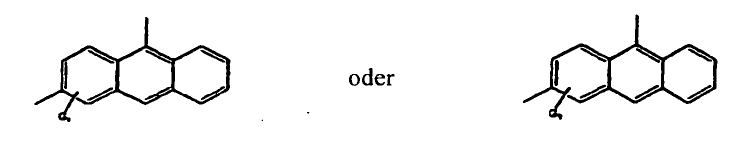 darstellt, worin G₁ -H; ein(en) C₂₋₁₀, gerad- oder verzweigtkettig, Alkyl, Alkenyl, Alkynyl, Alkoxy, oder Aliphaten; C₃₋₁₀-Alicyclus; Einzel- oder Mehrringaromaten oder -aryl; Aryl-substituiertes Alkyl, Alkenyl oder Alkynyl; Einzel- oder Mehrringaryl-substituierten Aliphaten; Aliphat-substituierten Einzel- oder Mehrringaromaten; Akyl-, Alkenyl- oder Alkynyl-substituiertes Aryl; einen Einzel- oder Mehrringheterocyclus; einen Einzel-oder Mehrringheterocyclus-substituierten Aliphaten; einen Aliphat-substituierten Aromaten; Heterocyclus-substituiertes Alkyl, Alkenyl oder Alkynyl; Alkyl-, Akenyl- oder Alkynyl-substituierten Heterocyclus; oder Kombinationen davon darstellt.

5. Verwendung nach Anspruch 4, worin G₁ Folgendes darstellt: -H, -CH₃, -CH₂CH₃, -CH₂Ph, -NH₂, -NHCOCH₃, -N₃, -CN, -F, -CL, -BR, -I, -CF₃, -OCH₃, -OCH₂Ph, -COCH₃, -COOH, -COOCH₃, -COOCH₂CH₃, -COOCH₂Ph, -OCH₂CH₂O-, -C(NH₂)=NH oder Kombinationen davon.

6. Verwendung nach einem der Ansprüche 1 bis 5, weiter umfassend einen Substituenten an einer oder mehr von genannten Aminogruppen oder an einem oder mehr benachbarten Kohlenstoffatom(en).

7. Verwendung nach Anspruch 6, worin genanntes eine oder mehr benachbarte Kohlenstoffatom(e) eines oder beide der terminalen Kohlenstoffatome in Spermin darstellen.

8. Verwendung nach Anspruch 6 oder 7, worin genannter Substituent ein(en) C₁₋₁₀, gerad- oder vezweigtkettig, Alkyl, Alkenyl, Alkynyl, Alkoxy, oder Aliphaten; C₃₋₁₀-Alicyclus; Einzel- oder Mehrringaromaten oder -aryl; Aryl-substituiertes Alkyl, Alkenyl oder Alkynyl; Einzel- oder Mehrringaryl-substituierten Aliphaten; Aliphat-substituierten Einzel- oder Mehrringaromaten; Akyl-, Alkenyl- oder Alkynyl-substituiertes Aryl; einen Einzel- oder Mehrringheterocyclus; einen Einzel- oder Mehrringheterocyclus-substituierten Aliphaten; einen Aliphat-substituierten Aromaten; Heterocyclus-substituiertes Alkyl, Alkenyl oder Alkynyl; oder Alkyl-, Akenyl-, oder Alkynyl-substituierten Heterocyclus darstellt.

9. Verwendung nach Anspruch 8, worin genannter Substituent Methyl, Ethyl, Phenyl oder CH₂Ph darstellt.

10. Verwendung nach Anspruch 9, worin genanntes Analogon 1283 darstellt.

11. Verwendung eines Polyamin-Analogons von Spermidin bei der Herstellung eines Arzneimittels zur Induktion von Expression des Antizyms voller Länge,
worin genanntes Polyamin-Analogon die Expression eines Antizyms voller Länge induziert und Folgendes umfasst:
drei Amingruppen, die zur Bildung von drei positiven Ladungen bei physiologischem pH fähig sind, worin die ersten und zweiten Amingruppen durch den Abstand von fünf C-C- und/oder C-N-Bindungen getrennt sind und die genannten zweiten und dritten Amingruppen durch den Abstand von vier C-C und/oder C-N-Bindungen getrennt sind.

12. Verwendung nach Anspruch 11, weiter umfassend einen Substituenten an einer oder mehr von genannten Amingruppe(n) oder an einem oder mehr benachbarten Kohlenstoffatom(en).

13. Verwendung nach Anspruch 12, worin genanntes eine oder mehr benachbarte Kohlenstoffatom(e) eines oder beide der terminalen Kohlenstoffatome in Spermidin darstellen.

14. Verwendung nach Anspruch 12, worin genannter Substituent ein Carbonsäureamid oder Sulfonamid-Amid an der N¹-Stellung von Spermidin darstellt.

15. Verwendung nach Anspruch 14, worin genanntes Carbonsäureamid einen Alkyl- oder Arylcarbonsäure-Substituenten darstellt, umfassend ein(en) C₂₋₁₀, gerad- oder vezweigtkettig, Alkyl, Alkenyl, Alkynyl, Alkoxy oder Aliphaten; C₃₋₁₀-Alicyclus; Einzel-oder Mehrringaromaten oder -aryl; Aryl-substituiertes Alkyl, Alkenyl oder Alkynyl; Einzel- oder Mehrringaryl-substituierten Aliphaten; Aliphat-substituierten Einzel- oder Mehrringaromaten; Akyl-, Alkenyl- oder Alkynyl-substituiertes Aryl; einen Einzel- oder Mehrringheterocyclus; einen Einzel- oder Mehrringheterocyclus-substituierten Aliphaten; einen Aliphat-substituierten Aromaten; Heterocyclus-substituiertes Alkyl, Alkenyl oder Alkynyl; oder Alkyl-, Akenyl- oder Alkynyl-substituierten Heterocyclus.

16. Verwendung nach Anspruch 14, worin genanntes Sulfonamid-Amid einen Alkyl- oder Arylsulfonamid-Substituenten darstellt, umfassend ein(en) C₂₋₁₀, gerad- oder verzweigtkettig, Alkyl, Alkenyl, Alkynyl, Alkoxy oder Aliphaten; C₃₋₁₀-Alicyclus; Einzel- oder Mehrringaromaten oder -aryl; Aryl-substituiertes Alkyl, Alkenyl oder Alkynyl; Einzel- oder Mehrringaryl-substituierten Aliphaten; Aliphat-substituierten Einzel- oder Mehrringaromaten; Akyl-, Alkenyl- oder Alkynyl-substituiertes Aryl; einen Einzel- oder Mehrringheterocyclus; einen Einzel- oder Mehrringheterocyclus-substituierten Aliphaten; einen Aliphat-substituierten Aromaten; Heterocyclus-substituiertes Alkyl, Alkenyl oder Alkynyl; oder Alkyl-, Akenyl- oder Alkynyl-substituierten Heterocyclus.

17. Verwendung nach Anspruch 15 oder 16, worin genannter Alkyl-enthaltender Substituent weiter ein Halogenid enthält oder genannte Alkoxy-Komponente Methoxy, Ethoxy und substituiertes oder nicht substituiertes Benzyloxy darstellt.

18. Verwendung nach Anspruch 15, worin genanntes Analogon 1041 darstellt.

19. Verwendung eines Polyamin-Analogons von Putrescin bei der Herstellung eines Arzneimittels zur Induktion der Expression eines Antizyms voller Länge,
worin genanntes Polyamin-Analogon die Expression eines Antizyms voller Länge induziert, nicht Agmatin darstellt und Folgendes umfasst:
zwei Amingruppen, die zur Bildung von zwei positiven Ladungen bei physiologischem pH fähig sind, die durch den Abstand von fünf C-C-, C-O-, N-O- und/oder C-N-Bindungen oder mehr getrennt sind.

20. Verwendung nach Anspruch 19, weiter umfassend einen oder mehr Substituenten an einer oder mehr der genannten Amingruppen oder an einem oder mehr benachbarten Kohlenstoffatom(en).

21. Verwendung nach Anspruch 20, worin genannter eine oder mehr Substituent(en) Folgendes darstellt/darstellen: ein(en) C₁₋₁₀, gerad- oder vezweigtkettig, Alkyl, Alkenyl, Alkynyl, Alkoxy, oder Aliphaten; C₃₋₁₀-Alicyclus; Einzel- oder Mehrringaromaten oder -aryl; Aryl-substituiertes Alkyl, Alkenyl oder Alkynyl; Einzel- oder Mehrringaryl-substituierten Aliphaten; Aliphat-substituierten Einzel- oder Mehrringaromaten; Alkyl-, Alkenyl- oder Alkynyl-substituiertes Aryl; einen Einzel- oder Mehrringheterocyclus; einen Einzel- oder Mehrringheterocyclus-substituierten Aliphaten; einen Aliphat-substituierten Aromaten; Heterocyclus-substituiertes Alkyl, Alkenyl oder Alkynyl; oder Alkyl-, Akenyl- oder Alkynyl-substituierten Heterocyclus.

22. Verwendung nach Anspruch 21, worin genannter eine oder mehr Substituent(en) -H, -CH₃, -CH₂CH₃ und -CH₂CH₂- darstellt/darstellen.

23. Verwendung nach Anspruch 22, worin genanntes Analogon aus Folgendem ausgewählt ist: 1,4-Diaminobutan mit Monoethyl- oder Dimethylsubstituenten in der 1-, 2-, 3- oder 4-Stellung; 1,4-, 1,3- oder 1,2-Dimethyldiaminobutan; Bis-1,4-cyclopropyl-1,4-diaminobutan; und 1,1,4,4-Tetramethyldiaminobutan.

24. Verwendung nach Anspruch 21, worin genanntes Analogon 1354 darstellt.

25. Verwendung nach einem der vorangehenden Ansprüche, worin genanntes Arzneimittel weiter einen pharmazeutisch verträglichen Hilfsstoff umfasst.

26. In-vitro-Verfahren zur Induktion der Expression eines Antizyms voller Länge, umfassend das Kontaktieren eines Antizym-Expressionssystems mit einem Analogon von jedwedem einen der vorangehenden Ansprüche.

27. In-Vitro-Verfahren zum Inhibieren des Zellwachstums, umfassend das Kontaktieren der genannten Zelle mit einem Analogon nach einem der vorangehenden Ansprüche.

## Revendications

1. Utilisation d'un analogue polyaminé de la spermine dans la préparation d'un médicament destiné à induire l'expression d'une antizyme à longueur intégrale
dans laquelle ledit analogue polyaminé induit l'expression d'une antizyme à longueur intégrale et comprend
quatre groupements amine susceptibles de former quatre charges positives à pH physiologique, dans lesquels les premier et deuxième groupements amine, et les troisième et quatrième groupements amine, sont séparés par la distance de quatre liaisons C-C et/ou C-N et lesdits deuxième et troisième groupements amine sont séparés par la distance de cinq ou plusieurs liaisons C-C et/ou C-N.

2. Utilisation selon la revendication 1, dans laquelle lesdits deuxième et troisième groupements amine sont séparés par -CH₂-Groupement-CH₂-
dans lequel ledit Groupement est un alkyle, alcényle, alcynyle, alcoxy, ou aliphatique, en C₂₋₁₀, linéaire ou ramifié ; alicyclique en C₃₋₁₀ ; aromatique ou aryle mono- ou polycyclique ; alkyle, alcényle ou alcynyle substitué par aryle ; aliphatique substitué par aryle mono- ou polycyclique; aromatique mono- ou polycyclique substitué par aliphatique ; aryle substitué par alkyle, alcényle ou alcynyle ; hétérocyclique mono- ou polycyclique ; aliphatique substitué par hétérocyclique mono- ou polycyclique ; aromatique substitué par aliphatique ; alkyle, alcényle ou alcynyle substitué par hétérocyclique ; ou hétérocyclique substitué par alkyle, alcényle ou alcynyle.

3. Utilisation selon la revendication 2, dans laquelle ledit Groupement est un aromatique ou aryle mono- ou polycyclique ; aromatique mono- ou polycyclique substitué par aliphatique ; aryle substitué par alkyle, alcényle ou alcynyle ; hétérocyclique mono- ou polycyclique ; aromatique substitué par aliphatique ; ou hétérocyclique substitué par alkyle, alcényle ou alcynyle.

4. Utilisation selon la revendication 3, dans laquelle ledit Groupement est l'un quelconque parmi les groupements suivants : 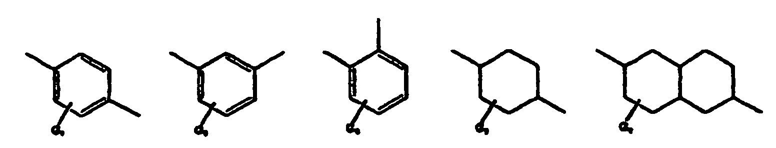 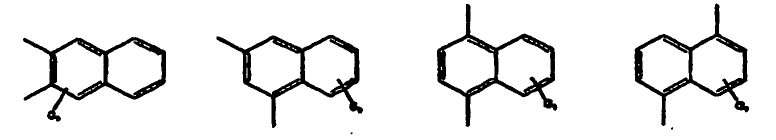 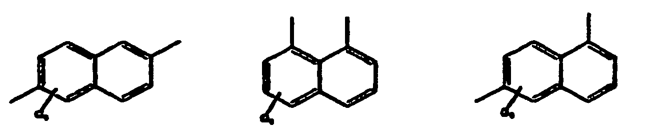 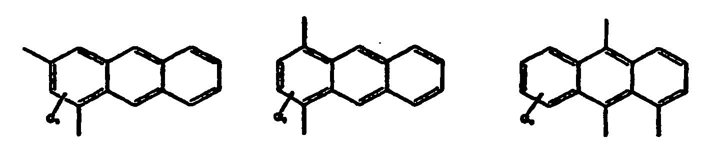 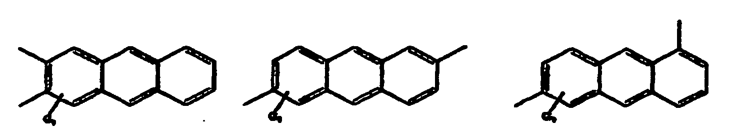 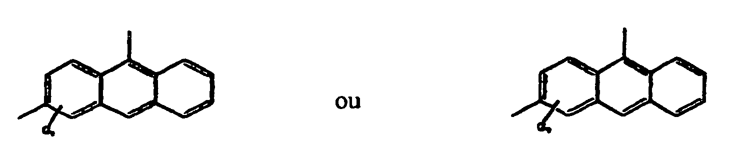 dans lesquels G₁ est -H ; alkyle, alcényle, alcynyle, alcoxy, ou aliphatique, en C₂₋₁₀, linéaire ou ramifié ; alicyclique en C₃₋₁₀ ; aromatique ou aryle mono- ou polycyclique ; alkyle, alcényle ou alcynyle substitué par aryle ; aliphatique substitué par aryle mono- ou polycyclique ; aromatique mono- ou polycyclique substitué par aliphatique ; aryle substitué par alkyle, alcényle ou alcynyle ; hétérocyclique mono- ou polycyclique ; aliphatique substitué par hétérocyclique mono- ou polycyclique ; aromatique substitué par aliphatique ; alkyle, alcényle ou alcynyle substitué par hétérocyclique ; hétérocyclique substitué par alkyle, alcényle ou alcynyle ; ou des associations de ceux-ci.

5. Utilisation selon la revendication 4, dans laquelle G₁ est -H, -CH₃, -CH₂CH₃, -CH₂Ph, -NH₂, -NHCOCH₃, -N₃, -CN, -F, -CL, -BR, -I, -CF₃, -OCH₃, -OCH₂Ph, -COCH₃, -COOH, -COOCH₃, -COOCH₂CH₃, -COOCH₂Ph, -OCH₂CH₂O-, -C(NH₂)=NH, ou des associations de ceux-ci.

6. Utilisation selon l'une quelconque des revendications 1 à 5, comprenant en outre un substituant sur un ou plusieurs desdits groupements amine ou sur un ou plusieurs atomes de carbone adjacents.

7. Utilisation selon la revendication 6, dans laquelle ledit ou lesdits un ou plusieurs atome(s) de carbone adjacent(s) est (sont) l'un des atomes de carbone terminaux de la spermine, ou les deux.

8. Utilisation selon la revendication 6 ou 7, dans laquelle ledit substituant est un alkyle, alcényle, alcynyle, alcoxy, ou aliphatique, en C₁₋₁₀, linéaire ou ramifié ; alicyclique en C₃₋₁₀ ; aromatique ou aryle mono- ou polycyclique ; alkyle, alcényle ou alcynyle substitué par aryle ; aliphatique substitué par aryle mono- ou polycyclique ; aromatique mono- ou polycyclique substitué par aliphatique ; aryle substitué par alkyle, alcényle ou alcynyle ; hétérocyclique mono- ou polycyclique ; aliphatique substitué par hétérocyclique mono- ou polycyclique ; aromatique substitué par aliphatique ; alkyle, alcényle ou alcynyle substitué par hétérocyclique ; ou hétérocyclique substitué par alkyle, alcényle ou alcynyle.

9. Utilisation selon la revendication 8, dans laquelle ledit substituant est méthyle, éthyle, phényle ou CH₂Ph.

10. Utilisation selon la revendication 9, dans laquelle ledit analogue est 1283.

11. Utilisation d'un analogue polyaminé de la spermidine dans la préparation d'un médicament destiné à induire l'expression d'une antizyme à longueur intégrale
dans laquelle ledit analogue polyaminé induit l'expression d'une antizyme à longueur intégrale et comprend
trois groupements amine susceptibles de former trois charges positives à pH physiologique, dans lesquels les premier et deuxième groupements amine sont séparés par la distance de cinq liaisons C-C et/ou C-N et lesdits deuxième et troisième groupements amine sont séparés par la distance de quatre liaisons C-C et/ou C-N.

12. Utilisation selon la revendication 11, comprenant en outre un substituant sur un ou plusieurs desdits groupements amine ou sur un ou plusieurs atomes de carbone adjacents.

13. Utilisation selon la revendication 12, dans laquelle ledit ou lesdits un ou plusieurs atome(s) de carbone adjacent(s) est (sont) l'un des atomes de carbone terminaux de la spermidine, ou les deux.

14. Utilisation selon la revendication 12, dans laquelle ledit substituant est un amide carboxylique ou amide de sulfonamide sur la position N¹ de la spermidine.

15. Utilisation selon la revendication 14, dans laquelle ledit amide carboxylique est un substituant alkyl- ou aryl-carboxylique comprenant un alkyle, alcényle, alcynyle, alcoxy, ou aliphatique, en C₂₋₁₀, linéaire ou ramifié ; alicyclique en C₃₋₁₀ ; aromatique ou aryle mono- ou polycyclique ; alkyle, alcényle ou alcynyle substitué par aryle ; aliphatique substitué par aryle mono- ou polycyclique ; aromatique mono- ou polycyclique substitué par aliphatique ; aryle substitué par alkyle, alcényle ou alcynyle ; hétérocyclique mono- ou polycyclique ; aliphatique substitué par hétérocyclique mono- ou polycyclique ; aromatique substitué par aliphatique ; alkyle, alcényle ou alcynyle substitué par hétérocyclique ; ou hétérocyclique substitué par alkyle, alcényle ou alcynyle.

16. Utilisation selon la revendication 14, dans laquelle ledit amide de sulfonamide est un substituant alkyl- ou aryl-sulfonamide comprenant un alkyle, alcényle, alcynyle, alcoxy, ou aliphatique, en C₂₋₁₀, linéaire ou ramifié ; alicyclique en C₃₋₁₀ ; aromatique ou aryle mono- ou polycyclique ; alkyle, alcényle ou alcynyle substitué par aryle ; aliphatique substitué par aryle mono- ou polycyclique ; aromatique mono- ou polycyclique substitué par aliphatique ; aryle substitué par alkyle, alcényle ou alcynyle ; hétérocyclique mono- ou polycyclique ; aliphatique substitué par hétérocyclique mono- ou polycyclique ; aromatique substitué par aliphatique ; alkyle, alcényle ou alcynyle substitué par hétérocyclique ; ou hétérocyclique substitué par alkyle, alcényle ou alcynyle.

17. Utilisation selon la revendication 15 ou 16, dans laquelle ledit substituant alkylé contient en outre un halogénure, ou ledit motif alcoxy est méthoxy, éthoxy, et benzyloxy substitué ou non substitué.

18. Utilisation selon la revendication 15, dans laquelle ledit analogue est 1041.

19. Utilisation d'un analogue polyaminé de la putréscine dans la préparation d'un médicament destiné à induire l'expression d'une antizyme à longueur intégrale
dans laquelle ledit analogue polyaminé induit l'expression d'une antizyme à longueur intégrale, n'est pas l'agmatine, et comprend
deux groupements amine susceptibles de former deux charges positives à pH physiologique, séparés par la distance de cinq ou plusieurs liaisons C-C, C-O, N-O et/ou C-N.

20. Utilisation selon la revendication 19, comprenant en outre un ou plusieurs substituant(s) sur un ou plusieurs desdits groupements amine ou sur un ou plusieurs atomes de carbone adjacents.

21. Utilisation selon la revendication 20, dans laquelle ledit ou lesdits un ou plusieurs substituant(s) est (sont) un alkyle, alcényle, alcynyle, alcoxy, ou aliphatique, en C₁₋₁₀, linéaire ou ramifié ; alicyclique en C₃₋₁₀ ; aromatique ou aryle mono- ou polycyclique ; alkyle, alcényle ou alcynyle substitué par aryle ; aliphatique substitué par aryle mono- ou polycyclique ; aromatique mono- ou polycyclique substitué par aliphatique ; aryle substitué par alkyle, alcényle ou alcynyle ; hétérocyclique mono- ou polycyclique ; aliphatique substitué par hétérocyclique mono- ou polycyclique ; aromatique substitué par aliphatique ; alkyle, alcényle ou alcynyle substitué par hétérocyclique ; ou hétérocyclique substitué par alkyle, alcényle ou alcynyle.

22. Utilisation selon la revendication 21, dans laquelle ledit ou lesdits un ou plusieurs substituant(s) est (sont) -H, -CH₃, -CH₂CH₃, et -CH₂CH₂-.

23. Utilisation selon la revendication 22, dans laquelle ledit analogue est choisi parmi le 1,4-diaminobutane comportant des substituants monométhyle ou diméthyle en position 1, 2, 3 ou 4 ; le 1,4-, 1,3-, ou 1,2-diméthyl-diaminobutane ; le bis-1,4-cyclopropyl-1,4-diaminobutane ; et le 1,1,4,4-tétraméthyldiaminobutane.

24. Utilisation selon la revendication 21, dans laquelle ledit analogue est 1354.

25. Utilisation selon l'une quelconque des revendications précédentes, dans laquelle ledit médicament comprend en outre un excipient pharmaceutiquement acceptable.

26. Méthode in vitro d'induction de l'expression d'une antizyme à longueur intégrale, comprenant la mise en contact d'un système d'expression d'une antizyme avec un analogue selon l'une quelconque des revendications précédentes.

27. Méthode in vitro d'inhibition de la croissance cellulaire, comprenant la mise en contact de ladite cellule avec un analogue selon l'une quelconque des revendications précédentes.
